# EUROPEAN PATENT APPLICATION

(11) **EP 4 218 819 A2**
(43) Date of publication of application: **02.08.2023**
(21) Application number: 23158416.0
(22) Date of filing: 05.12.2016
(51) Int. Cl.: A61K 45/06, A61K 31/045, A61K 31/06, A61K 31/122, A61K 31/275, A61K 31/327, A61K 31/53, A61K 31/661, A61K 39/395, A61P 31/00, A61P 31/04, A61P 31/06, A61P 31/08, A61P 31/10, A61P 31/12, A61P 31/14, A61P 31/16, A61P 31/18, A61P 31/20, A61P 31/22, A61P 35/00

(54) **COMBINATION THERAPY BASED ON PD-1 SIGNAL INHIBITORS**

(30) Priority: 07.12.2015 JP 2015238511; 16.06.2016 JP 2016119695
(62) Divisional of application: 16872932.5
(71) Applicant: Kyoto University, Kyoto-shi, Kyoto 606-8501 (JP)
(72) Inventor: HONJO, Tasuku, Kyoto-shi, 606-8501 (JP); CHAMOTO, Kenji, Kyoto-shi, 606-8501 (JP); FAGARASAN, Sidonia, Yokohama-shi, 230-0045 (JP)
(74) Representative: Mewburn Ellis LLP

(57) **Abstract**

A novel therapeutic strategy for the anti-PD-1 antibody therapy is provided.

A pharmaceutical composition which comprises at least one substance selected from the group consisting of the following (i) to (iii) and is administered before, after or simultaneously with the administration of a PD-1 signal inhibitor:
(i) ROS generators and substances that regulate downstream signaling pathways thereof,
(ii) substances exhibiting an uncoupling effect and substances that regulate downstream signaling pathways thereof, and
(iii) amino acids.

## Description

### TECHNICAL FIELD

The present invention relates to a combination therapy using PD-1 signal inhibitors.

### BACKGROUND ART

The results of recent clinical trials have revealed that the anti-PD-1 antibody therapy is more effective than conventional standard therapies in various cancers (Non-Patent Documents Nos. 1-3). The response rate of PD-1 antibody therapy in terminal lung cancer patients was 20-30%, showing a dramatic improvement compared to conventional anti-cancer drug therapies. However, only about one half of the patients were non-responsive. Little is known about why those patients are non-responsive to the PD-1 antibody therapy.

### PRIOR ART LITERATURE

### Non-Patent Documents

Non-Patent Document No. 1: Bramer J, Reckamp K, et al: Nivolumab versus Docetaxel in Advanced Nonsquamous Non-Small-Cell Lung Cancer. N Engl J Med, 373:1627-1639, 2015
Non-Patent Document No. 2: Hamanishi J, Mandai M, Ikeda T, et al: Safety and Antitumor Activity of Anti-PD-1 Antibody, Nivolumab, in Patients with Platinum-Resistant Ovarian Cancer. J Clin Oncol, 33:4015-4022, 2015
Non-Patent Document No. 3: Motzer RJ, Escudier B, McDermott DF, et al: Nivolumab versus Everolimus in Advanced Renal-Cell Carcinoma. N Engl J Med, 373:1803-1813, 2015

### DISCLOSURE OF THE INVENTION

### PROBLEM FOR SOLUTION BY THE INVENTION

It is an object of the present invention to provide a novel therapeutic strategy for anti-PD-1 antibody therapy.

### MEANS TO SOLVE THE PROBLEM

Unlike the conventional anti-cancer drugs which have a direct killing effect on cancer cells, anti-PD-1 antibody therapy inhibits cancer proliferation by activating antitumor immunity. It is expected that antitumor effect can be improved by a combined use of anti-PD-1 antibody and reagents which support antitumor immunity. Such a combination therapy may be applicable to non-responsive patients.

The present inventors have found that antitumor effect is synergistically improved when PD-1 signal inhibiting antibodies are used in combination with ROS generators or mitochondrial membrane potential regulators (uncouplers). ROS generators or mitochondrial membrane potential regulators alone exhibited no antitumor effect in vivo. From these results, it is demonstrated that ROS generators or mitochondrial membrane potential regulators support antitumor immunity and synergistically inhibit cancer proliferation when used in combination with PD-1 signal inhibitory antibody.

A summary of the present invention is as described below.
(1) A pharmaceutical composition which comprises at least one substance selected from the group consisting of the following (i) to (iii) and is administered before, after or simultaneously with the administration of a PD-1 signal inhibitor:
   (i) ROS generators and substances that regulate downstream signaling pathways thereof,
   (ii) substances exhibiting an uncoupling effect and substances that regulate downstream signaling pathways thereof, and
   (iii) amino acids.
(2) The pharmaceutical composition of (1) above, wherein the PD-1 signal inhibitor is an antibody.
(3) The pharmaceutical composition of (1) or (2) above, wherein the antibody is at least one antibody selected from the group consisting of anti-PD-1 antibody, anti-PD-L1 antibody and anti-PD-L2 antibody.
(4) The pharmaceutical composition of any one of (1) to (3) above, wherein the ROS generator is at least one compound selected from the group consisting of tert-butyl hydroperoxide, carbonyl cyanide p-trifluoromethoxyphenylhydrazone, 2,4-dinitrophenol, 2,3-dimethoxy-1, 4-naphthoquinone and analogs thereof.
(5) The pharmaceutical composition of any one of (1) to (3) above, wherein the substance exhibiting an uncoupling effect is at least one compound selected from the group consisting of carbonyl cyanide p-trifluoromethoxyphenylhydrazone, 2,4-dinitrophenol, carbonyl cyanide m-chlorophenylhydrazone, salicylic acid, 4,4'-[pentane-1,5-diylbis(oxy)]dibenzenecarboximidamide, 2-(2-(2,6-dichlorophenylamino)phenyl)acetic acid, 4-hydroxy-2-methyl-N-(2-pyridinyl)-2H-1,2-benzothiazine-3-carboxamide 1,1-dioxide, 2- {1-[(4-chlorophenyl)carbonyl]-5-methoxy-2-methyl- 1H-indol-3-yl} acetic acid, N-(4-nitro-2-phenoxyphenyl)methanesulfonamide, 4-hydroxy-2-methyl-N-(5-methyl-2-thiazolyl)-2H-1,2-benzothiazine-3-carboxamide-1,1-diox ide, niclosamide ethanolamine salt, 3-methylbut-2-enyl 4-methoxy-8-(3-methylbut-2-enyloxy)quinoline-2-carboxylate and analogs thereof.
(6) The pharmaceutical composition of any one of (1) to (3) above, wherein the substance that regulates downstream signaling pathways of ROS generators or substances exhibiting an uncoupling effect is a substance which regulates one or more of mTOR, AMPK, SIRT1, PGC-1α/transcription factor complex (PGC-1α-comprising transcription factor complex) and Foxo1.
(7) The pharmaceutical composition of (6) above, wherein the substance that regulates mTOR is at least one compound selected from the group consisting of 4,6-di-4-morpholinyl-N-(4-nitrophenyl)-1,3,5-triazin-2-amine, phosphatidic acid and analogs thereof.
(8) The pharmaceutical composition of (6) above, wherein the substance that regulates AMPK is at least one compound selected from the group consisting of 6,7-dihydro-4-hydroxy-3-(2'-hydroxy[1,1'-biphenyl]-4-yl)-6-oxo-thieno[2,3-b]pyridine-5-car bonitrile, 5-aminoimidazole-4-carboxamide 1-β-D-ribofuranoside, N,N-dimethylimidodicarbonimidic diamide, 6-[4-[2-(1-piperidinyl)ethoxy]phenyl]-3-(4-pyridinyl)pyrazolo[1,5-a]pyrimidine and analogs thereof.
(9) The pharmaceutical composition of (6) above, wherein the substance that regulates SIRT1 is at least one compound selected from the group consisting of trans-3,5,4'-trihydroxystilbene, N-(2-(3-(piperazin-1-ylmethyl)imidazo[2,1-b]thiazol-6-yl)phenyl)quinoxaline-2-carboxamide, N-benzyl-3,5-dicarbethoxy-4-phenyl-1,4-dihydropyridine, 2-amino-N-cyclopentyl-1-(3-methoxypropyl)-1H-pyrrolo[2,3-b]quinoxaline-3-carboxamide, nicotinamide mononucleotide and analogs thereof.
(10) The pharmaceutical composition of (6) above, wherein the substance that regulates PGC-1α/transcription factor complex (transcriptional factor complexes comprising PGC-1 α ) is at least one compound selected from the group consisting of 2-(4- {2-[(4-chlorobenzoyl)amino]ethyl}phenoxy)-2-methylpropanoic acid, 9-cis,12-cis-octadecadienoic acid, 2-[4-(4-chlorobenzoyl)phenoxy]-2-methyl-propanoic acid-d6 1-methylethyl ester, (undecylthio)-acetic acid, 4-methyl-5 -(2-pyrazinyl)-3 -dithio lethione, N,N-dimethylformamide, 3-[4-(2,4-bis-trifluoromethylbenzyloxy)-3-methoxyphenyl]-2-cyano-n-(5-trifluoromethyl-1,3, 4-thiadiazol-2-yl)acrylamide and analogs thereof.
(11) The pharmaceutical composition of (6) above, wherein the substance that regulates Foxo1 is at least one compound selected from the group consisting of 5-amino-7-(cyclohexylamino)-1-ethyl-6-fluoro-4-oxo-1,4-dihydroquinoline-3-carboxylic acid, 2-cyclopentyl-N-{2,4-dichloro-3-[(isoquinolin-5-yloxy)methyl]phenyl}-N-methylacetamide and analogs thereof.
(12) The pharmaceutical composition of any one of (1) to (3) above, wherein the amino acid is at least one compound selected from the group consisting of tryptophan, phenylalanine, leucine, isoleucine, tyrosine, histidine, lysine, methionine, threonine, valine, alanine, arginine, asparagine, aspartic acid, cysteine, glutamic acid, glutamine, glycine, proline, serine, ornithine, citrulline and analogs thereof.
(13) The pharmaceutical composition of any one of (1) to (12) above, which is used as an anti-cancer agent, an anti-infective agent or a combination thereof.
(14) The pharmaceutical composition of any one of (1) to (13) above, wherein the PD-1 signal inhibitor is administered separately from the at least one substance selected from the group consisting of the following (i) to (iii):
   (i) ROS generators and substances that regulate downstream signaling pathways thereof,
   (ii) substances exhibiting an uncoupling effect and substances that regulate downstream signaling pathways thereof, and
   (iii) amino acids.
(15) The pharmaceutical composition of any one of (1) to (13) above, which is a combination drug comprising the PD-1 signal inhibitor and the at least one substance selected from the group consisting of the following (i) to (iii):
   (i) ROS generators and substances that regulate downstream signaling pathways thereof,
   (ii) substances exhibiting an uncoupling effect and substances that regulate downstream signaling pathways thereof, and
   (iii) amino acids.
(16) A drug which enhances PD-1 signal inhibitory activity, comprising at least one substance selected from the group consisting of the following (i) to (iii):
   (i) ROS generators and substances that regulate downstream signaling pathways thereof,
   (ii) substances exhibiting an uncoupling effect and substances that regulate downstream signaling pathways thereof, and
   (iii) amino acids.
(17) A method of treating cancer, infection or a combination thereof, comprising administering to a human or animal subject a pharmaceutically effective amount of at least one substance selected from the group consisting of the following (i) to (iii) before, after or simultaneously with the administration of a PD-1 signal inhibitor:
   (i) ROS generators and substances that regulate downstream signaling pathways thereof,
   (ii) substances exhibiting an uncoupling effect and substances that regulate downstream signaling pathways thereof, and
   (iii) amino acids.
(18) Use of at least one substance selected from the group consisting of the following (i) to (iii) for treating cancer, infection or a combination thereof, wherein the at least one substance selected is administered before, after or simultaneously with the administration of a PD-1 signal inhibitor:
   (i) ROS generators and substances that regulate downstream signaling pathways thereof,
   (ii) substances exhibiting an uncoupling effect and substances that regulate downstream signaling pathways thereof, and
   (iii) amino acids.
(19) Use of at least one substance selected from the group consisting of the following (i) to (iii) in a method for treating cancer, infection or a combination thereof, wherein the at least one substance selected is administered:
   (i) ROS generators and substances that regulate downstream signaling pathways thereof,
   (ii) substances exhibiting an uncoupling effect and substances that regulate downstream signaling pathways thereof, and
   (iii) amino acids.

### EFFECT OF THE INVENTION

Antitumor effect increases synergistically by using ROS generators or substances that regulate downstream signaling pathways thereof and/or substances exhibiting an uncoupling effect or substances that regulate downstream signaling pathways thereof in combination with PD-1 signal inhibitors.

The present specification encompasses the contents disclosed in the specification and/or drawings of Japanese Patent Applications Nos. 2015-238511 and 2016-119695 based on which the present application claims priority.

### BRIEF DESCRIPTION OF THE DRAWINGS

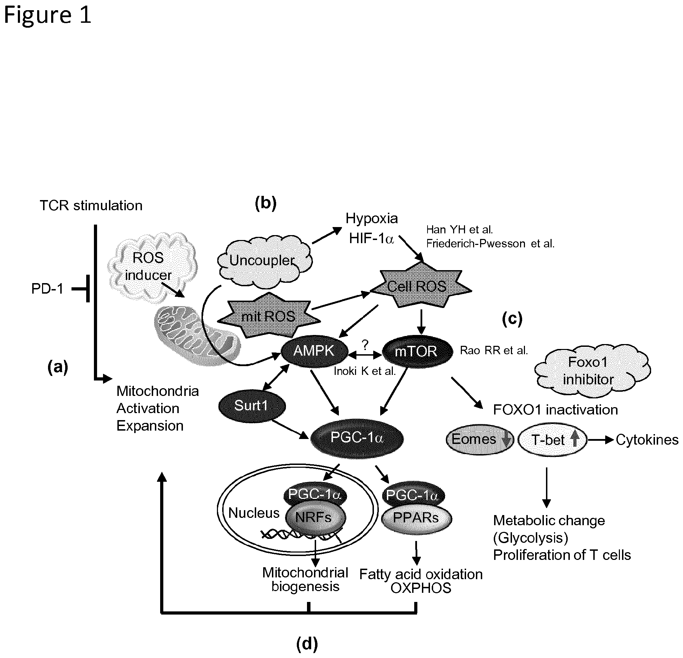

Fig. 1. Hypothetical scheme for mitochondrial activation by PD-1 blockade and chemical reagents.
   a) PD-1 blockade activates mitochondria of tumor-reactive T cells. b) ROS generators or uncouplers increase cellular ROS. c) Cellular ROS activates AMPK and mTOR, resulting in the activation of PGC-1α as well as the expression of T-bet. d) Activation of NRFs and PPARs which bind to PGC-1α induces feed-forward activation of mitochondria. Friederich-Persson, M., et al. Kidney hypoxia, attributable to increased oxygen consumption, induces nephropathy independently of hyperglycemia and oxidative stress. Hypertension 62, 914-919 (2013).
   Kenwood, B.M., et al. Identification of a novel mitochondrial uncoupler that does not depolarize the plasma membrane. Mol Metab 3, 114-123 (2014). Berrien-Elliott, M.M., et al. Checkpoint blockade immunotherapy relies on T-bet but not Eomes to induce effector function in tumor-infiltrating CD8+ T cells. Cancer immunology research 3, 116-124 (2015).

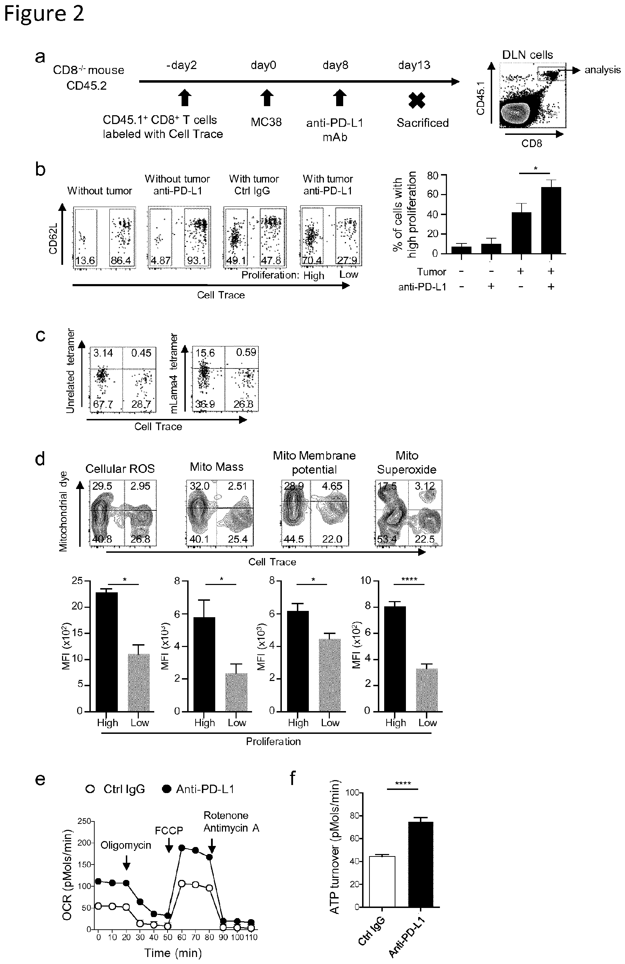

Fig. 2. In vivo detection of TR CTLs by PD-1 blockade and mitochondrial activity therein.
   a-d) Schematic diagrams of the experimental steps. CellTrace-labeled CD45.1⁺ CD8⁺ T cells were transferred into CD45.2⁺ CD8^{-/-} mice. The mice were inoculated with MC38 and treated with PD-L1 antibody. CD8⁺ CD45.1⁺ T cells in draining lymph nodes were gated and analyzed (a). Intensities of CD62L and CellTrace in cells in the gate are shown (Left) (b). The frequencies of highly proliferating cells were compared between groups. Data represent the means ± SEM of four or five mice. *p < 0.05, one-way ANOVA analysis (Right) (b). Among the gated cells shown in (a), the positivity of MHC tetramer loaded with CellTrace and mLama4 peptide or an unrelated peptide was analyzed in the group treated with PD-L1 antibody (c). Draining lymph node cells treated with PD-L1 antibody were stained with dyes indicating mitochondrial activity. Representative FACS data of the gated population in (a) are shown (Upper) (d). The median of fluorescence intensity (MFI) of each dye was compared between highly proliferating (high) and less proliferating (low) populations. Data represent the means ± SEM of five mice. *p< 0.05, ****p< 0.0001, one-way ANOVA analysis (Lower) (d). Data are representative of three independent experiments (a-d). e, f) MC38-bearing wild-type mice were treated with PD-L1 antibody every 4 days for a total of 3 times. The oxygen consumption rate (OCR) of draining lymph node CD8⁺ T cells isolated from treated or untreated mice was measured by XF^{e}96 analyzer. Two days after the second treatment, cells from three mice were mixed and analyzed (e). ATP turnover defined as (last measured value after oligomycin injection) - (lowest measured value after oligomycin injection) was calculated (f). Data represent the means ± SEM of three wells. ****p < 0.0001, two-sided Student t test distribution. Data are representative of two independent experiments.

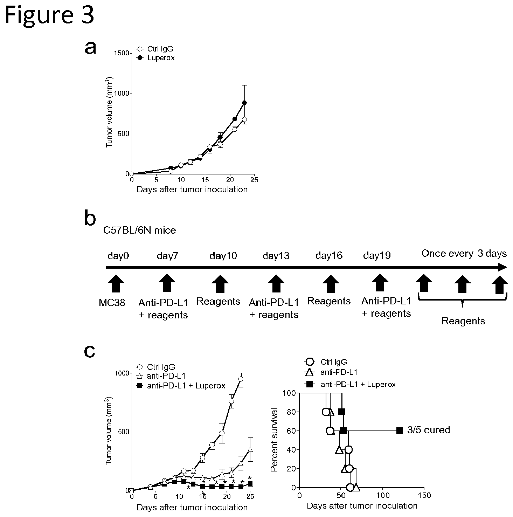

Fig. 3. Synergistic effect of Luperox, a ROS generator, with PD-L1 antibody therapy. a) MC38 was inoculated into mice. Then, for the following 3 weeks, tert-butyl hydroperoxide solution (Luperox) was administered to the mice every 7 [Office1] days. Tumor volumes are shown. Data represent the means ± SEM of five mice. b) Schematic diagram of the combination therapy schedule, c) Seven days after inoculation of MC38, the mice were treated with PD-L1 antibody and Luperox. Tumor volumes and survival curves are shown. Data represent the means ± SEM of five mice. *p<0.05, **p<0.01, two-sided Student t test distribution (anti-PD-L1 vs. anti-PD-L1 + Luperox). Data are representative of two independent experiments.

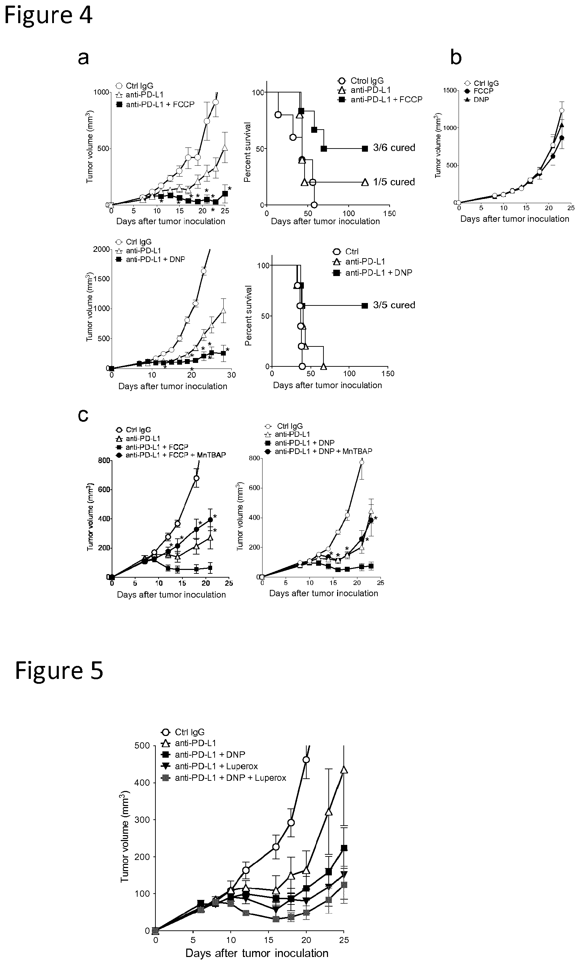

Fig. 4. The synergistic effect of uncouplers is mediated by ROS. a) MC38-bearing mice were treated with PD-L1 antibody in combination with FCCP or DNP according to the same schedule as shown in Fig. 3b. Tumor volumes and survival curves are shown. Data represent the means ± SEM of five or six mice. *p < 0.05, **p < 0.01, two-sided Student t test distribution (anti-PD-L1 vs. anti-PD-L1 + FCCP or DNP). b) MC38-bearing mice were treated with FCCP or DNP alone according to the same schedule as in a). Tumor volumes are shown. Data represent the means ± SEM of five mice. c) MC38-bearing mice were treated with a ROS scavenger (MnTBAP) and PD-L1 antibody in combination with FCCP (Left) or DNP (Right). Data represent the means ± SEM of four or five mice. *p < 0.05, **p < 0.01, two-sided Student t test distribution (combination therapy vs. combination therapy + MnTBAP). The mice of the control IgG group in DNP combination therapy (Right) were shared with those of Fig. 4b. Data are representative of two independent experiments.
Fig. 5. Antitumor effect is enhanced by using both DNP and Luperox. MC38-bearing mice were treated with PD-L1 antibody in combination with either DNP or Luperox or both according to the same schedule as shown in Fig. 3b. Tumor volumes are shown. Data represent the means ± SEM of five or six mice. Data are representative of two independent experiments.

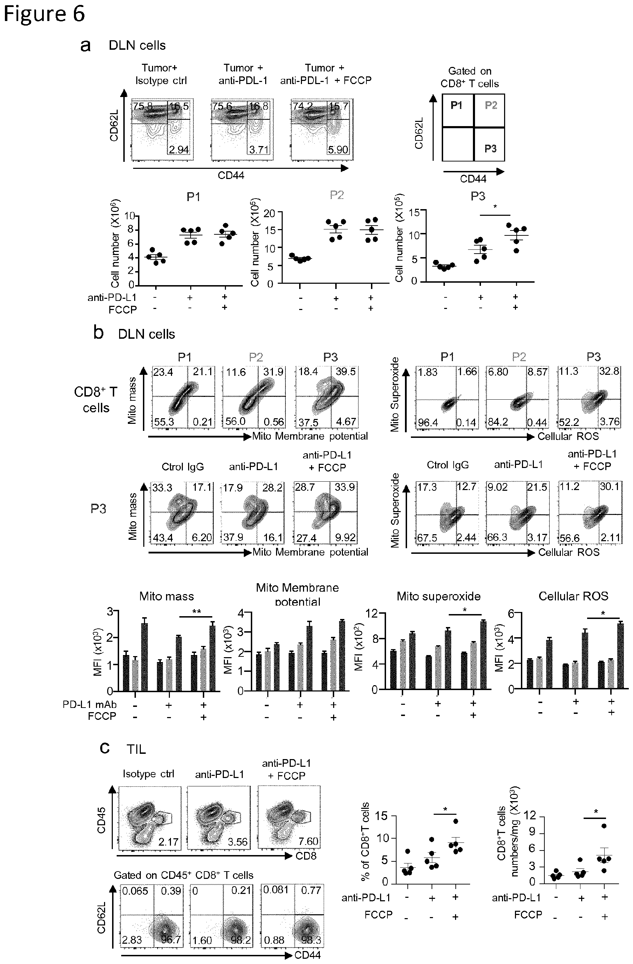

Fig. 6. Increase in the number of effector CD8⁺ T cells by FCCP administration, a) MC38-bearing mice were treated with PD-L1 antibody and FCCP according to the same schedule as shown in Fig. 3b. Draining lymph node (DLN) cells on day 14 were stained with anti-CD8, -CD62L, and -CD44 antibodies, and then CD8⁺ T cells were gated. P1 to P3 populations were defined based on the intensities of CD62L and CD44 (Upper). The absolute cell numbers of P1 to P3 in each group were calculated. Data represent the means ± SEM of five mice. *p < 0.05, one-way ANOVA analysis (Lower). b) DLN CD8⁺ T cells of mice treated with PD-L1 antibody and FCCP were stained with each mitochondrial dye. Representative FACS profiles of P1 to P3 (Upper). Representative FACS profiles of the P3 population stained with each mitochondrial dye in each group (Middle). MFI of P1 to P3 stained with each dye was compared between treated groups. Colors correspond to the P1 to P3 populations. Data represent the means ± SEM of five mice. *p <0.05, **p < 0.01, one-way ANOVA analysis (Lower). c) Cells isolated from tumor tissue which was enzymatically treated on day 11 of tumor inoculation were stained with anti-CD8, -CD45, -CD62L, and
   -CD44 antibodies. CD45⁺ CD8⁺ T cells were gated, and their CD62L and CD44 phenotypes were analyzed (Left). The frequencies of CD8⁺ T cells in CD45⁺ T cells and the absolute numbers of CD45⁺ CD8⁺ T cells were compared between groups (Right). Data represent the means ± SEM of five mice. *p< 0.05, **p<0.01, one-way ANOVA analysis. FACS data are representative of five mice in each group. Data are representative of two independent experiments.

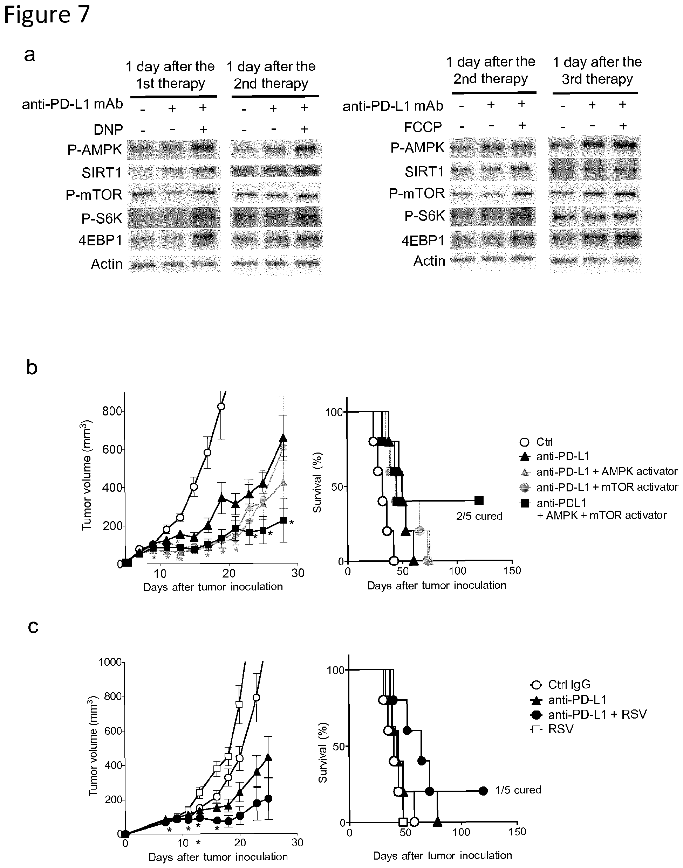

Fig. 7. Both mTOR and AMPK pathways are involved in the synergistic effect of the uncouplers and PD-L1 antibody combination, a) In the combination therapy using DNP or FCCP, draining lymph node cells from five mice were mixed, and CD8⁺ T cells were isolated. Phosphorylation of AMPK, ACC, mTOR and S6K and the expression of SIRT1 and 4EBP1 were analyzed by Western blotting, b) MC38-bearing mice were treated with PD-L1 antibody in combination with AMPK activator (A769662) and/or mTOR activator (MHY1485) according to the same schedule as shown in Fig. 3b. Tumor volumes and survival curves are shown. Data represent the means ± SEM of five mice. *p < 0.05, **p < 0.01, two-sided Student t test distribution (anti-PD-L1 antibody vs. combination therapy). Each color of asterisk corresponds to the group indicated by the same color. c) MC38-bearing mice were treated with an SIRT1 activator (Resveratrol) and PD-L1 antibody. Tumor volumes and survival curves are shown. Data represent the means ± SEM of five mice. *p< 0.05, **p< 0.01, two-sided Student t test distribution (anti-PD-L1 antibody vs. combination therapy).

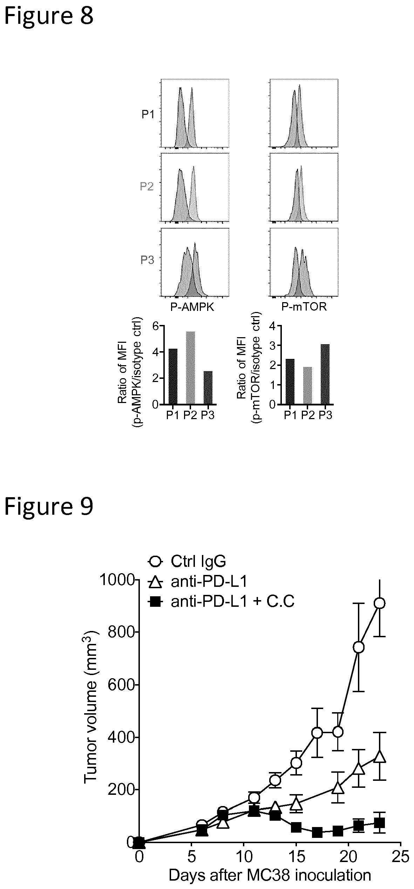

Fig. 8. Different activated states of mTOR and AMPK correspond to the differentiation stages of CD8⁺ T cells. MC38-bearing mice were treated with DNP and PD-L1 antibody. One day after the first therapy, draining lymph node cells were stained with antibodies against CD8, CD62L, CD44, p-mTOR and p-AMPK. After gating on P1 to P3 as shown in Fig. 6a, the fluorescence intensities of p-AMPK and p-mTOR were compared. Data are representative of two independent experiments.
Fig. 9. Synergistic effect of PD-L1 antibody therapy and AMPK inhibitor. MC38-bearing mice were treated with compound C and anti-PD-L1 antibody according to the same schedule as shown in Fig. 3b. Tumor volumes are shown.

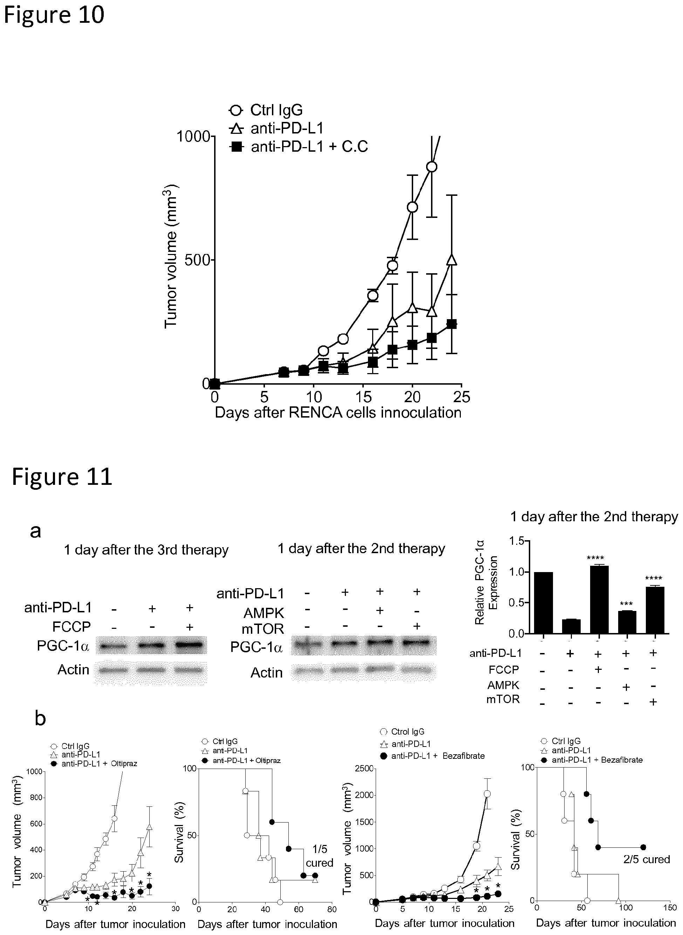

Fig. 10. Synergistic effect of PD-L1 antibody therapy and AMPK inhibitor. RENCA-bearing mice were treated with compound C and anti-PD-L1 antibody according to the same schedule as shown in Fig. 3b. Tumor volumes are shown.
Fig. 11. Synergistic effect of PD-L1 antibody therapy and PGC-1α/transcription factor activator. MC38-bearing mice were treated with anti-PD-L1 antibody in combination with NRF2 activator (oltipraz) or PPARs activator (bezafibrate) according to the same schedule as shown in Fig. 3b. Tumor volumes are shown. Data represent the means ± SEM of five mice. *p< 0.05, two-tailed Student t test (anti-PD-L1 antibody vs. each combination therapy).

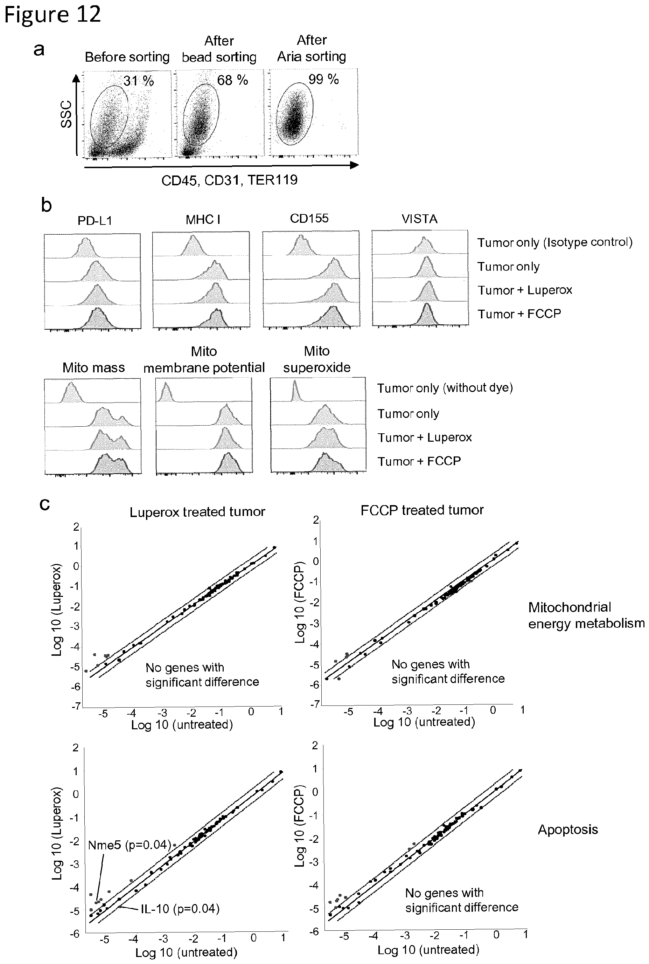

Fig. 12. Luperox and FCCP have little effect on tumor cells *in vivo.* a) Tumor tissues were harvested one day after the second injection of FCCP or Luperox alone. After enzymatic digestion, tumor cells were sorted with a cell isolation kit capable of excluding non-tumor cells. The isolated tumor cells were further purified with Aria and used in the following experiments. b) Tumor cells were stained with antibodies targeting PD-L1, MHC class I, CD155 (ligand of TIGIT), and VISTA or with dyes for mitochondrial mass, membrane potential, and superoxide. c) Expression levels of genes associated with mitochondrial energy metabolism and apoptosis were examined using RT² profiler PCR Kit. Indicated genes were those among the 82 genes tested which were significantly up-regulated more than twofold compared with untreated tumor cells.

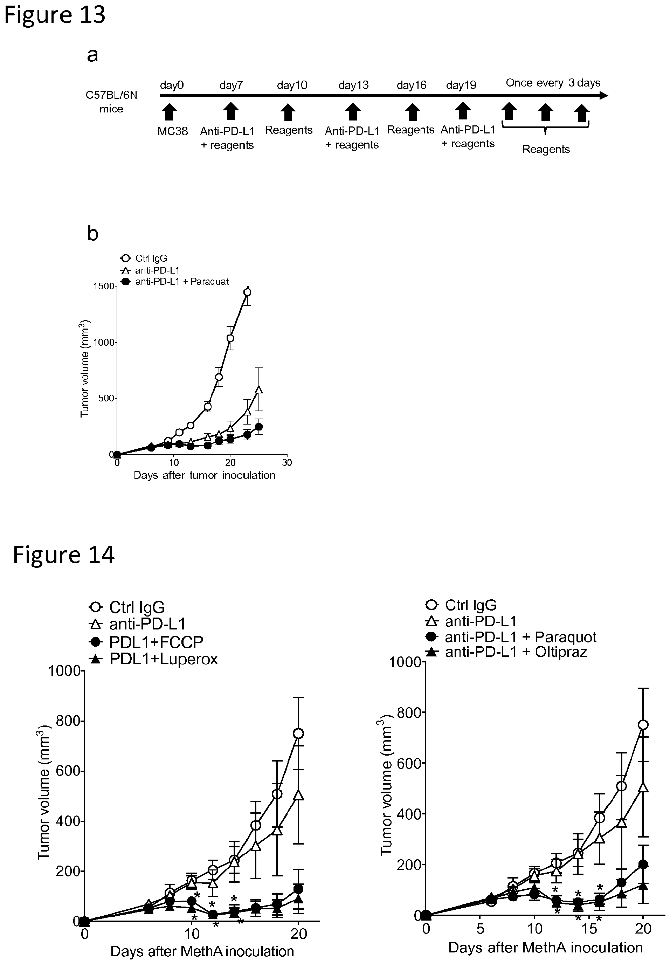

Fig. 13. Antitumor synergistic effects of mitochondrial activation-associated reagents, a) Schematic diagram for a combinational therapy schedule, b) Mice were treated with PD-L1 antibody and paraquat in combination. Data represent the means ± SEM of five or six mice. Data are representative of two independent experiments.
Fig. 14. Mitochondrial activation-related reagents work on even different tumors and mouse strains. Fibrosarcoma MethA-transplanted BALB/c mice were treated with PD-L1 antibody in combination with FCCP, Luperox, paraquat or oltipraz according to the schedule shown in Fig. 3. Tumor sizes are shown. Data represent the means ± SEM of five mice. *p<0.05, two-tailed Student t test (anti-PD-L1 antibody vs. each combination therapy).

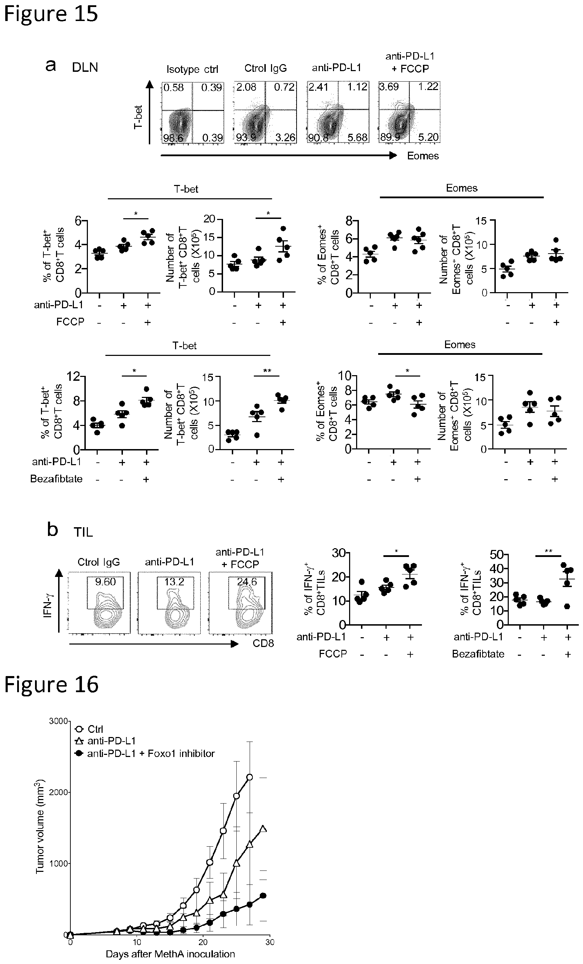

Fig. 15. T-bet and IFN-γ productions are up-regulated in combination therapy groups using PD-L1 antibody along with FCCP or bezafibrate. a) T-bet and Eomes expressions were analyzed by flow cytometry in DLN CD8⁺ T cells from mice treated with both PD-L1 antibody and FCCP. Representative FACS data are shown (Upper). The frequencies and absolute numbers of T-bet⁺ and Eomes⁺ T cells were calculated in DLN cells from mice treated with both PD-L1 antibody and FCCP or bezafibrate (Lower). b) Enzymatically digested tumor tissues were incubated at 37 °C for 6 hr, and IFN-γ was intracellularly stained in the CD8⁺ T cells from mice treated with both PD-L1 antibody and FCCP or bezafibrate. Representative FACS data (Left) and the frequency of IFN-γ⁺ cells in CD8⁺ T cells are shown (Right). Data represent the means ± SEM of five mice. *p< 0.05, **p<0.01, one-way ANOVA.
Fig. 16. Synergistic effect of PD-L1 antibody therapy and Foxol inhibitor: AS1842856. BALB/c mice were inoculated with MethA (5 × 10⁵). After 7 days, the mice were treated with PD-L1 antibody (80 µg) and Foxo1 inhibitor (2 mg/kg). Administration schedule was as shown in Fig. 3. Tumor volumes are shown.

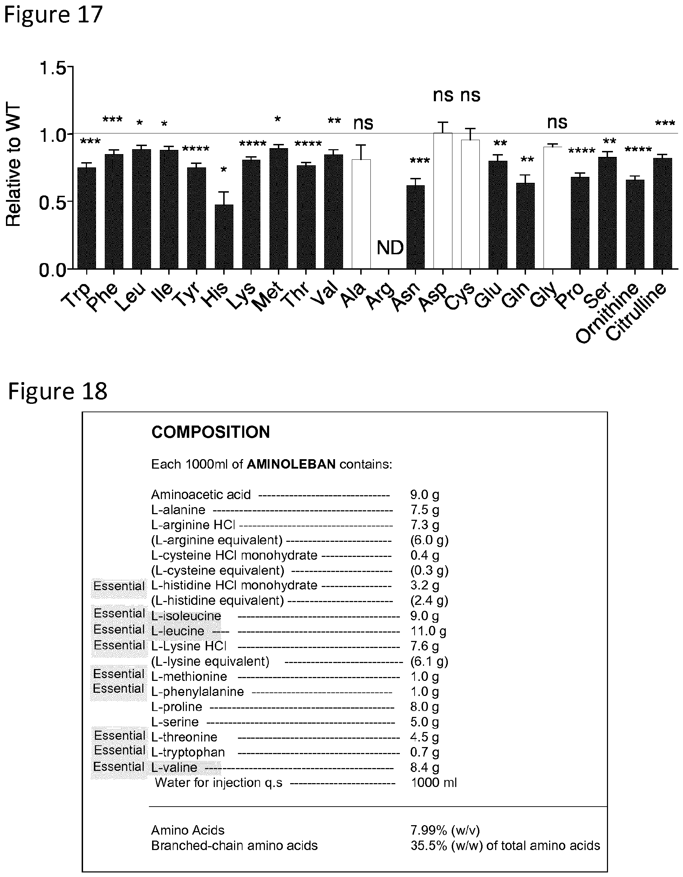

Fig. 17. Changes in blood metabolites (amino acids). Levels of amino acids contained in the sera of five each of PD-1^{-/-} and wild type mice were determined by GC-MS analysis. Shown in the graph are values for PD-1^{-/-} mice, with the corresponding values for wild type mice being taken as 1. N=5, *p<0.05, **p<0.01, ***p<0.001, ****p<0.0001, t-test.
Fig. 18. Composition of Aminoleban

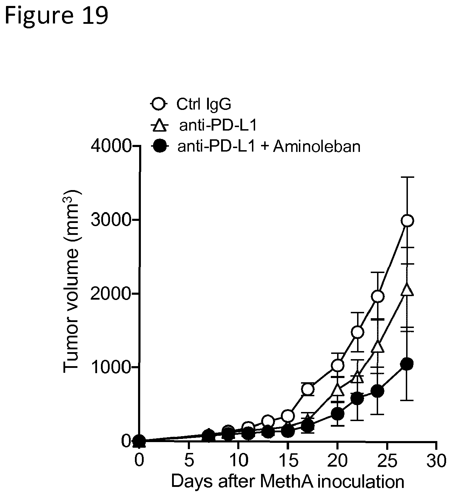

Fig. 19. Synergistic effect of PD-L1 antibody therapy combined with Aminoleban. BALB/c mice were inoculated with MethA (5 × 10⁵). After 7 days, the mice were treated with anti-PD-L1 antibody (60 µg) and Aminoleban (400 µl/mouse). Administration followed the schedule shown in Fig. 3.

### BEST MODES FOR CARRYING OUT THE INVENTION

Hereinbelow, the present invention will be described more specifically.

The present invention provides a pharmaceutical composition which comprises at least one substance selected from the group consisting of the following (i) to (iii) and is administered before, after or simultaneously with the administration of a PD-1 signal inhibitor:
(i) ROS generators and substances that regulate downstream signaling pathways thereof,
(ii) substances exhibiting an uncoupling effect and substances that regulate downstream signaling pathways thereof, and
(iii) amino acids.

Specific examples of ROS generators include, but are not limited to, tert-butyl hydroperoxide (Luperox), carbonyl cyanide p-trifluoromethoxyphenylhydrazone (FCCP), 2,4-dinitrophenol (DNP), 2,3-dimethoxy-1, 4-naphthoquinone (DMNQ), 2-methylnaphthalene-1,4-dione (Menadione), 1,1'-dimethyl-4,4'-bipyridinium dichloride (paraquat) and analogs thereof.

Downstream of ROS generators, there are signaling pathways for mTOR, ATM (ataxia telangiectasia mutated protein), AMPK (AMP-activated protein kinase) and so forth (see Fig. 1). The pharmaceutical composition of the present invention may comprise a substance which regulates any of these substances. The term "regulate" is a concept encompassing activation and inactivation.

mTOR, a downstream signaling pathway of ROS, activates cellular glycolysis, causing amplification of proteins, lipids and nucleic acids necessary for cell division (promotion of anabolic action). By this effect, the T cell receptor signal enhanced by the inhibition of PD-1 signal would be further intensified. When AMPK is activated as a downstream signaling pathway of uncouplers, mitochondrial biogenesis and oxidative phosphorylation necessary for energy production are activated in mitochondria. This would further augments the T cell receptor signal which is already enhanced by PD-1 signal inhibition.

Examples of substances which regulate mTOR include, but are not limited to, 4,6-dimorpholino-N-(4-nitrophenyl)-1,3,5-triazin-2-amine;4,6-Di-4-morpholinyl-N-(4-nitrop henyl)-1,3,5-triazin-2-amine (MHY1485), phosphatidic acid (PA) and analogs thereof. These substances activate mTOR.

AMPK, also known as an energy sensor, is activated when energy consumption leads to a decreased ATP level and an increased AMP level. AMPK plays a role in controlling metabolism as the energy is not consumed but restored. For this reason, when AMPK is activated, biosynthesis is inhibited (inhibition of anabolism) and ATP is actively produced from mitochondria (promotion of catabolism)^{1,2,3}. In the downstream of AMPK signaling pathway, a complex of PGC-1α and transcription factors exist, which activates mitochondrial biogenesis and oxidative phosphorylation metabolism ⁴. Since activation of AMPK is accompanied by amelioration of diabetes, some AMPK activators are used as therapeutics for diabetes. As substances which regulate AMPK, 6,7-dihydro-4-hydroxy-3-(2'-hydroxy[1,1'-biphenyl]-4-yl)-6-oxo-thieno[2,3-b]pyridine-5-car bonitrile (A769662), 5-aminoimidazole-4-carboxamide 1-β-D-ribofuranoside (AICAR), N,N-dimethylimidodicarbonimidic diamide (Metformin) and analogs thereof may be enumerated. These substances activate AMPK. As substances which inactivate AMPK (AMPK inhibitors), 6-[4-[2-(1-Piperidinyl)ethoxy]phenyl]-3-(4-pyridinyl)pyrazolo[1,5-a]pyrimidine (compound C) and analogs thereof may be enumerated.

SIRT1 is a deacetylase and activates transcription-related factors such as PGC-1α and Foxo1. Once activated, SIRT1 forms tolerance to ROS or stress. SIRT1 is also activated by AMPK, and promotes mitochondrial biogenesis and oxidative phosphorylation via activation of PGC-1α. Since tolerance to outer/inner stress is formed via activation of SIRT1, this enzyme is also reported to be associated with longevity^{5,6}. As substances which regulate SIRT1, trans-3,5,4'-trihydroxystilbene (resveratrol), N-(2-(3-(piperazin-1-ylmethyl)imidazo[2,1-b]thiazol-6-yl)phenyl)quinoxaline-2-carboxamide, N-benzyl-3,5-dicarbethoxy-4-phenyl-1,4-dihydropyridine, 2-amino-N-cyclopentyl-1-(3-methoxypropyl)-1H-pyrrolo[2,3-b]quinoxaline-3-carboxamide, nicotinamide mononucleotide and analogs thereof may be enumerated. These substances activate SIRT1.

PGC-1α, which is called a transcription factor coactivator, forms complexes with transcription factors related to various mitochondrial activities (transcription factor complexes comprising PGC-1α). For example, PGC-1α forms complexes with transcription factors TRβ1, NRFs, ERR_{S}, PPARα/β/^{™}[MOU2], etc. and promotes activation of mitochondria, facilitation of lipid metabolism, and detoxication of reactive oxygen species. Drugs targeting those transcription factors that bind to PGC-1α are used as therapeutics for diabetes and triglyceride lowering drugs^{4,7,8}. Specific examples of substances which regulate PGC-1α/transcription factor complexes (transcription factor complexes comprising PGC-1α) include, but are not limited to, 2-(4- {2-[(4-chlorobenzoyl)amino]ethyl}phenoxy)-2-methylpropanoic acid (bezafibrate), 9-cis,12-cis-octadecadienoic acid, 2-[4-(4-chlorobenzoyl)phenoxy]-2-methyl-propanoic acid-d6 1-methylethyl ester, (undecylthio)-acetic acid, 4-methyl-5-(2-pyrazinyl)-3-dithiolethion (oltipraz), N,N-dimethylformamide, 3-[4-(2,4-bis-trifluoromethylbenzyloxy)-3-methoxyphenyl]-2-cyano-n-(5-trifluoromethyl-1,3, 4-thiadiazol-2-yl)acrylamide and analogs thereof. These substances regulate PGC-1α/transcription factor complexes (transcription factor complexes comprising PGC-1α).

As substances exhibiting an uncoupling effect, carbonyl cyanide p-trifluoromethoxyphenylhydrazone (FCCP), 2,4-dinitrophenol (DNP), carbonyl cyanide m-chlorophenylhydrazpne (CCCP), salicylic acid, 4,4'-[pentane-1,5-diylbis(oxy)]dibenzenecarboximidamide) (Pentamidine), 2-(2-(2,6-dichlorophenylamino)phenyl)acetic acid (Diclofenac), 4-hydroxy-2-methyl-N-(2-pyridyl)-2H-1,2-benzothiazine-3-carboxamide 1,1-dioxide (Piroxicam), 2-{1-[(4-Chlorophenyl)carbonyl]-5-methoxy-2-methyl-1H-indol-3-yl} acetic acid (Indomethacin), (N-(4-nitro-2-phenoxyphenyl)methanesulfonamide (Nimesulide), 4-hydroxy-2-methyl-N-(5-methyl-2-thiazolyl)-2H-1,2-benzothiazine-3-carboxamide 1,1-dioxide (Meloxicam), niclosamide ethanolamine salt (NEN), 3-Methylbut-2-enyl 4-methoxy-8-(3-methylbut-2-enyloxy)quinoline-2-carboxylate and analogs thereof may be enumerated.

In the downstream of uncouplers, there are signal transductions such as mTOR, AMPK (AMP-activated protein kinase), SIRT1, PGC-1α/transcription factor complex (transcription factor complexes comprising PGC-1α) and so forth (see Fig. 1). The pharmaceutical composition of the present invention may comprise a substance which regulates any of these substances mentioned above. The term "regulate" is a concept encompassing activation and inactivation.

The substances which regulate mTOR, AMPK, SIRT1 and PGC-1α/transcription factor complex (PGC-1α-comprising transcription factor complex) are as described above.

Foxo1 is a transcription factor located in the downstream of mTOR signal. When phosphorylated via activation of mTOR, Foxo1 translocates from the nucleus to the cytoplasm, and does not initiate transcription. As a result, EOMES is inhibited and expression of T-bet necessary for CTL activation is enhanced (Staron MM et al. Immunity. 41: 802-14, 2014; Rao RR et al. 36: 374-87, 2012).

Moreover, Foxo1 binds to PGC-1α to thereby activate gene expression which promotes gluconeogenesis. In gluconeogenesis, glucose is synthesized from metabolites of TCA cycle, so oxidative phosphorylation tends to be inhibited. It has also been reported that inhibition of Foxo1 in type 2 diabetes model results in a recovery from mitochondrial dysfunction. This seems to be another evidence to show that inhibition of Foxo1 activates mitochondria (Coppari R et al. Nat Clin Pract Endocrinol Metab. 3:160-6, 2009; Puigserver P et al. Nature. 423:550-5, 2003).

As substances that regulate Foxo1, 5-amino-7-(cyclohexylamino)-1-ethyl-6-fluoro-4-oxo-1,4-dihydroquinoline-3-carboxylic acid, 2-cyclopentyl-N-{2,4-dichloro-3-[(isoquinolin-5-yloxy)methyl]phenyl}-N-methylacetamide, and analogs thereof may be enumerated.

As amino acids, tryptophan, phenylalanine, leucine, isoleucine, tyrosine, histidine, lysine, methionine, threonine, valine, alanine, arginine, asparagine, aspartic acid, cysteine, glutamic acid, glutamine, glycine, proline, serine, ornithine, citrulline and analogs thereof may be enumerated. Amino acids may be used either singly or in combinations of two or more kinds. Amino acid may be either L-amino acid, D-amino acid or DL-amino acid. When PD-1 is inhibited, anabolism (cell proliferation) undergo, resulting in the consumption of metabolites such as amino acids. If, under such circumstances, amino acids serving as the skeleton of cells, etc. are supplied, cell proliferation would be further promoted. When the supplied amino acids accelerate cell proliferation, mTOR could be activated. For example, an amino acid preparation Aminoleban has been reported to activate mTOR (Tamanna N, Mahmood N. Int Sch Res Notices. 2014:235619, 2014). In the examples to be described later, Aminoleban exhibited a synergistic effect when combined with PD-L1 antibody therapy, so the amino acids contained in Aminoleban would preferably be used either alone or in combinations of two or more kinds.

The substances described in (i) to (iii) above may take the form of salts. Such salts may be pharmaceutically acceptable salts. Examples of such salts include, but are not limited to, alkaline metal salts such as sodium salts, potassium salts or lithium salts; alkaline earth metal salts such as calcium salts or magnesium salts; metal salts such as aluminum salts, iron salts, zinc salts, copper salts, nickel salts or cobalt salts; amine salts including inorganic salts such as ammonium salts and organic salts such as t-octylamine salts, dibenzylamine salts, morpholine salts, glucosamine salts, phenylglycine alkyl ester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenethylamine salts, piperazine salts, tetramethylammonium salts or tris(hydroxymethyl)aminomethane salts; hydrohalogenic acid salts such as hydrofluorides, hydrochlorides, hydrobromides or hydroiodides; *inorganic acid salts such as nitrates,* perchlorates, sulfates or phosphates; lower alkane sulfonic acid salts such as methanesulfonates, trifluoromethanesulfonates or ethanesulfonates; arylsulfonic acid salts such as benzenesulfonates or p-toluenesulfonates; organic acid salts such as acetates, malates, fumarates, succinates, citrates, tartrates, oxalates or maleates; and amino acid salts such as glycine salts, lysine salts, arginine salts, ornithine salts, glutamic acid salts or aspartic acid salts. These salts may be prepared by known methods.

Moreover, the substances described in (i) to (iii) above and salts thereof may produce solvates with solvents such as water, methanol, ethanol or acetonitrile. Such solvates may be either of a single kind or a mixture of one or more kinds.

As used herein, the term "analog" refers to a substance which does not require a major structural change but involves only slight structural changes (such as addition or modification of side chains or the like) to exhibit an effect comparable to that of the original substance. "Analog" is a concept encompassing derivatives of lead compounds in pharmaceuticals, prodrugs to active metabolites, active metabolites to prodrugs, and so on. Examples of such prodrugs include, but are not limited to, compounds in which an amino group of the active compound is acylated, alkylated or phosphorylated (e.g., an amino acid of the active compound is eicosanoylated, alanylated, pentylaminocarbonylated, (5-methyl-2-oxo-1,3-dioxolen-4-yl)methoxycarbonylated, tetrahydrofuranylated, pyrrolidyl methylated, pivaloyloxymethylated, tert-butylated, or the like); compounds in which a hydroxyl group of the active compound is acylated, alkylated, phosphorylated or borated (e.g., a hydroxyl group in the active compound is acetylated, palmitoylated, propanoylated, pivaloylated, succinylated, fumarylated, alanylated, dimethylaminomethylcarbonylated, or the like); and compounds in which a carboxy group of the active compound is esterified or amidated (e.g., a carboxy group of the active compound is ethyl esterified, phenyl esterified, carboxymethyl esterified, dimethylaminomethyl esterified, pivaloyloxymethyl esterified, ethoxycarbonyloxyethyl esterified, phthalidyl esterified, (5-methyl-2-exo-1,3-dioxolen-4-yl)methyl esterified, cyclohexyloxycarbonylethyl esterified, methylamidated, or the like).

As used herein, the term "PD-1 signal" refers to the signal transduction mechanism which PD-1 bears. As one aspect of this mechanism, PD-1 inhibits T cell activation in collaboration with its ligands PD-L1 and PD-L2. PD-1 (Programmed cell death-1) is a membrane protein expressed in activated T cells and B cells. Its ligands PD-L1 and PD-L2 are expressed in various cells such as antigen-presenting cells (monocytes, dendritic cells, etc.) and cancer cells. PD-1, PD-L1 and PD-L2 work as inhibitory factors which inhibit T cell activation. Certain types of cancer cells and virus-infected cells escape from host immune surveillance by expressing the ligands of PD-1 to thereby inhibit T cell activation.

As PD-1 signal inhibitors, substances which specifically bind to PD-1, PD-L1 or PD-L2 may be given. Such substances include, but are not limited to, proteins, polypeptides, oligopeptides, nucleic acids (including natural-type and artificial nucleic acids), low molecular weight organic compounds, inorganic compounds, cell extracts, and extracts from animals, plants, soils or the like. These substances may be either natural or synthetic products. Preferable PD-1 signal inhibitors are antibodies. More preferably, antibodies such as anti-PD-1 antibody, anti-PD-L1 antibody and anti-PD-L2 antibody may be given. Any type of antibody may be used as long as it is capable of inhibiting the PD-1 signal. The antibody may be any of polyclonal antibody, monoclonal antibody, chimeric antibody, single chain antibody, humanized antibody or human-type antibody. Methods for preparing such antibodies are known. The antibody may be derived from any organisms such as human, mouse, rat, rabbit, goat or guinea pig. As used herein, the term "antibody" is a concept encompassing antibodies of smaller molecular sizes such as Fab, F(ab)'₂, ScFv, Diabody, V_{H}, V_{L}, Sc(Fv)₂, Bispecific sc(Fv)₂, Minibody, scFv-Fc monomer or scFv-Fc dimer.

The pharmaceutical composition of the present invention may be used as an anticancer agent, an anti-infective agent or a combination thereof.

When the pharmaceutical composition of the present invention is administered as an anticancer agent, target cancers or tumors includes, but are not limited to, leukemia, lymphoma (e.g., Hodgkin's disease, non-Hodgkin's lymphoma), multiple myeloma, brain tumors, breast cancer, endometrial cancer, cervical cancer, ovarian cancer, esophageal cancer, stomach cancer, appendix cancer, colon cancer, liver cancer, gallbladder cancer, bile duct cancer, pancreatic cancer, adrenal cancer, gastrointestinal stromal tumor, mesothelioma, head and neck cancer (such as laryngeal cancer), oral cancer (such as floor of mouth cancer), gingival cancer, tongue cancer, buccal mucosa cancer, salivary gland cancer, nasal sinus cancer (e.g., maxillary sinus cancer, frontal sinus cancer, ethmoid sinus cancer, sphenoid sinus cancer), thyroid cancer, renal cancer, lung cancer, osteosarcoma, prostate cancer, testicular tumor (testicular cancer), renal cell carcinoma, bladder cancer, rhabdomyosarcoma, skin cancer (e.g., basal cell cancer, squamous cell carcinoma, malignant melanoma, actinic keratosis, Bowen's disease, Paget's disease) and anal cancer.

When the pharmaceutical composition of the present invention is administered as an anti-infective agent, target infections include, but are not limited to, bacterial infections [various infections caused by Streptococcus (e.g., group A β hemolytic streptococcus, pneumococcus), Staphylococcus aureus (e.g., MSSA, MRSA), Staphylococcus epidermidis, Enterococcus, Listeria, Neisseria meningitis aureus, Neisseria gonorrhoeae, pathogenic Escherichia coli (e.g., 0157:H7), Klebsiella (Klebsiella pneumoniae), Proteus, Bordetella pertussis, Pseudomonas aeruginosa, Serratia, Citrobacter, Acinetobacter, Enterobacter, mycoplasma, Clostridium or the like; tuberculosis, cholera, plague, diphtheria, dysentery, scarlet fever, anthrax, syphilis, tetanus, leprosy, Legionella pneumonia (legionellosis), leptospirosis, Lyme disease, tularemia, Q fever, and the like], rickettsial infections (e.g., epidemic typhus, scrub typhus, Japanese spotted fever), chlamydial infections (e.g., trachoma, genital chlamydial infection, psittacosis), fungal infections (e.g., aspergillosis, candidiasis, cryptococcosis, trichophytosis, histoplasmosis, Pneumocystis pneumonia), parasitic protozoan infections (e.g., amoebic dysentery, malaria, toxoplasmosis, leishmaniasis, cryptosporidiosis), parasitic helminthic infections (e.g., echinococcosis, schistosomiasis japonica, filariasis, ascariasis, diphyllobothriasis latum), and viral infections [e.g., influenza, viral hepatitis, viral meningitis, acquired immune deficiency syndrome (AIDS), adult T-cell leukemia, Ebola hemorrhagic fever, yellow fever, cold syndrome, rabies, cytomegalovirus infection, severe acute respiratory syndrome (SARS), progressive multifocal leukoencephalopathy, chickenpox, herpes zoster, hand-foot-and-mouth disease, dengue, erythema infectiosum, infectious mononucleosis, smallpox, rubella, acute anterior poliomyelitis (polio), measles, pharyngoconjunctival fever (pool fever), Marburg hemorrhagic fever, hantavirus renal hemorrhagic fever, Lassa fever, mumps, West Nile fever, herpangina and chikungunya fever].

The pharmaceutical composition of the present invention comprises at least one substance selected from the group consisting of the following (i) to (iii) and is administered before, after or simultaneously with the administration of a PD-1 signal inhibitor:
(i) ROS generators and substances that regulate downstream signaling pathways thereof,
(ii) substances exhibiting an uncoupling effect and substances that regulate downstream signaling pathways thereof, and
(iii) amino acids.

In the pharmaceutical composition of the present invention, a PD-1 signal inhibitor and at least one substance selected from the group consisting of the above-mentioned (i) to (iii) may be used in combination or may be formulated as a single dosage.

When a PD-1 signal inhibitor and at least one substance selected from the group consisting of the above-mentioned (i) to (iii) are used in combination, the PD-1 signal inhibitor and the at least one substance may be administered separately.

When a PD-1 signal inhibitor and at least one substance selected from the group consisting of the above-mentioned (i) to (iii) are formulated as a single dosage, a combination drug containing the PD-1 signal inhibitor and the at least one substance may be prepared.

The pharmaceutical composition of the present invention is administered to human or animal subjects systemically or locally by an oral or parenteral route.

PD-1 signal inhibitors (e.g., anti-PD-1 antibody, anti-PD-L1 antibody or anti-PD-L2 antibody) may be dissolved in buffers such as PBS, physiological saline or sterile water, optionally filter- or otherwise sterilized before being administered to human or animal subjects by injection or infusion. To the solution of PD-1 signal inhibitors, additives such as coloring agents, emulsifiers, suspending agents, surfactants, solubilizers, stabilizers, preservatives, antioxidants, buffers, isotonizing agents and the like may be added. As routes of administration, intravenous, intramuscular, intraperitoneal, subcutaneous or intradermal administration and the like may be selected.

The content of the PD-1 signal inhibitor (e.g., anti-PD-1 antibody, anti-PD-L1 antibody or anti-PD-L2 antibody) in a preparation varies with the type of the preparation and is usually 1-100% by weight, preferably 50-100% by weight. Such a preparation may be formulated into a unit dosage form.

The dose and the number of times and frequency of administration of PD-1 signal inhibitor (e.g., anti-PD-1 antibody, anti-PD-L1 antibody or anti-PD-L2 antibody) may vary with the symptoms, age and body weight of the human or animal subject, the method of administration, dosage form and so on. For example, in terms of the amount of the active ingredient, 0.1-100 mg/kg body weight, preferably 1-10 mg/kg body weight, may usually be administered per adult at least once at a frequency that enables confirmation of the desired effect.

At least one substance selected from the group consisting of the above-described (i) to (iii) may be contained in a preparation comprising a PD-1 signal inhibitor. Alternatively, the at least one substance either alone or in admixture with an excipient or carrier may be formulated into tablets, capsules, powders, granules, liquids, syrups, aerosols, suppositories, injections or the like. The excipient or carrier may be of any type that is routinely used in the art and pharmaceutically acceptable, with their type and composition being appropriately changed. As a liquid carrier, for example, water, plant oil or the like may be used. As a solid carrier, saccharides such as lactose, sucrose or glucose, starches such as potato starch or corn starch, cellulose derivatives such as microcrystalline cellulose, and the like may be used. Lubricants such as magnesium stearate, binders such as gelatin or hydroxypropyl cellulose, and disintegrants such as carboxymethyl cellulose, and the like may be added. What is more, antioxidants, coloring agents, flavoring agents, preservatives, and the like may also be added.

At least one substance selected from the group consisting of the above-described (i) to (iii) may be administered via various routes such as oral, transnasal, rectal, transdermal, subcutaneous, intravenous or intramuscular route.

The content of at least one substance selected from the group consisting of the above-described (i) to (iii) in a preparation varies with the type of the preparation and.is usually 1-100% by weight, preferably 50-100% by weight. In the case of a liquid, for example, the content of the at least one substance selected from the group consisting of the above-described (i) to (iii) in the preparation is preferably 1-100% by weight. In the case of a capsule, tablet, granule or powder, the content of the at least one substance selected from the group consisting of the above-described (i) to (iii) in the preparation is usually 10-100% by weight, preferably 50-100% by weight, with the balance being the carrier. The preparation may be formulated into a unit dosage form.

The dose and the number of times and frequency of administration of at least one substance selected from the group consisting of the above-described (i) to (iii) may vary with the symptoms, age and body weight of the human or animal subject, the method of administration, dosage form and so on. For example, in terms of the amount of the active ingredient, 0.005 µg (or ml) to 25000 mg (or ml)/kg body weight may usually be administered per adult at least once at a frequency that enables confirmation of the desired effect. With respect to the dose of each drug (substance), values of the appropriate range, preferable range and more preferable range are shown in the table below, to which the dose is not necessarily limited, though.

**(Table)**

| | | Dose Appropriate range | Preferable range | Moe preferable range |
|---|---|---|---|---|
| ROS generator | | 0.5 ml/kg body weight - 25 ml/kg body weight | 5 ml/kg body weight - 2500 µl/kg body weight | 25 ml/kg body weight - 250 µl/kg body weight |
| Substance which regulates downstream signaling pathways of ROS generator | mTOR activator | 0.005 µg/kg body weight -1250 µg/kg body weight | 0.05 µg/kg body weight -125 µg/kg body weight | 0.5 µg/kg body weight -12.5 µg/kg body weight |
| Substance which regulates downstream signaling pathways of ROS generator | AMPK activator | 12.5 µg/kg body weight -3 g/kg body weight | 125 µg/kg body weight -300 mg/kg body weight | 1.25 mg/kg body weight -30 mg/kg body weight |
| Substance which regulates downstream signaling pathways of ROS generator | AMPK inhibitor | 20 µg/kg body weight - 5 g/kg body weight | 200 µg/kg body weight - 500 mg/kg body weight | 2 mg/kg body weight - 50 mg/kg body weight |
| Substance exhibiting an uncoupling effect | | 2.5 µg/kg body weight - 312.5 mg/kg body weight | 25 µg/kg body weight - 31.25 mg/kg body weight | 250 µg/kg body weight - 6.25 mg/kg body weight |
| Substance which regulates downstream signaling pathways of uncoupler | SIRT1 activator | 1 µg/kg body weight - 250 mg/kg body weight | 10 µg/kg body weight - 25 mg/kg body weight | 100 µg/kg body weight - 2.5 mg/kg body weight |
| Substance which regulates downstream signaling pathways of uncoupler | PGC-1α/ transcription factor complex activator | 1 µg/kg body weight - 250 mg/kg body weight | 10 µg/kg body weight - 25 mg/kg body weight | 100 µg/kg body weight - 2.5 mg/kg body weight |
| Amino acid | | 3.2 µg/kg body weight - 800 mg/kg body weight | 32 µg/kg body weight - 80 mg/kg body weight | 320 µg/kg body weight - 8 mg/kg body weight |

The ratio (in mass) of PD-1 signal inhibitor (e.g., anti-PD-1 antibody, anti-PD-L1 antibody or anti-PD-L2 antibody) to a ROS generator or a substance which regulates downstream signaling pathways thereof is appropriately from 1:0.1 to1:100, preferably from 1:1 to 1:50.

The ratio (in mass) of PD-1 signal inhibitor (e.g., anti-PD-1 antibody, anti-PD-L1 antibody or anti-PD-L2 antibody) to a substance exhibiting an uncoupling effect or a substance which regulates downstream signaling pathways thereof is appropriately from 1:0.01 to1:10, preferably from 1:0.1 to 1:1.

The ratio (in mass) of PD-1 signal inhibitor (e.g., anti-PD-1 antibody, anti-PD-L1 antibody or anti-PD-L2 antibody) to an amino acid(s) is appropriately from 1:0.001 to 1:100, preferably from 1:0.01 to 1:10.

The present invention also provides a method of treating cancer, infection or a combination thereof, comprising administering to a human or animal subject a pharmaceutically effective amount of at least one substance selected from the group consisting of the above-described (i) to (iii) before, after or simultaneously with the administration of a PD-1 signal inhibitor. Further, the present invention provides use of at least one substance selected from the group consisting of the above-described (i) to (iii) for treating cancer, infection or a combination thereof, wherein the at least one substance selected is administered before, after or simultaneously with the administration of a PD-1 signal inhibitor. Still further, the present invention provides use of at least one substance selected from the group consisting of the above-described (i) to (iii) in a method for treating cancer, infection or a combination thereof, wherein the at least one substance selected is administered before, after or simultaneously with the administration of a PD-1 signal inhibitor.

What is more, the present invention provides a drug which enhances PD-1 signal inhibitory activity, comprising at least one substance selected from the group consisting of the above-described (i) to (iii).

This drug of the present invention may be used in combination with a PD-1 signal inhibitor or formulated together with a PD-1 signal inhibitor into a combination drug. Combined use of a PD-1 signal inhibitor and at least one substance selected from the group consisting of the above-described (i) to (iii), as well as formulating them together as a single dosage are as described above. The drug of the present invention may be used as an experimental reagent in addition to its application as a pharmaceutical.

To examine whether a given substance is a ROS generator or not, the substance may be added to cells (e.g., mouse spleen cells) *in vitro,* followed by measurement of an intracellular ROS concentration. To examine whether or not a given substance has an uncoupling effect or an effect for regulating downstream signaling pathways thereof, the substance may be added to cells (e.g., mouse spleen cells) *in vitro,* followed by measurement of an intracellular mitochondrial proton gradient with a flow cytometer after intracellular staining or by direct measurement of the gradient with an analyzer Seahorse.

### EXAMPLES

Hereinbelow, the present invention will be described in more detail with reference to the following Examples.

### [Example 1]

### [Summary]

Cancer immunotherapy by PD-1 blockade has dramatically improved the survival rate of cancer patients. However, a certain percentage of patients are non-responsive to this therapy. A combination therapy of PD-1 blockade and other drugs is expected to improve the efficacy of immunotherapy. Using a mouse cancer therapy model, the present inventors have found that the tumor-reactive cytotoxic T lymphocytes (TR CTLs) occurring in draining lymph nodes have a large mitochondrial volume, a high membrane potential and high capability of reactive oxygen species (ROS) production. Moreover, the present inventors have demonstrated that mitochondria-derived ROS generated by ROS generator or uncouplers increases CD8⁺ CD44⁺ CD62L⁻ T cells in draining lymph nodes and tumors, thus enhancing the antitumor effect of PD-1 blockade. The present inventors have also revealed that reagents directly activating metabolic sensors such as mTOR, AMPK, SIRT, etc including Resveratrol enhance the therapeutic effect of PD-1 blockade and inhibit tumors for a long period of time. Importantly, none of these substances or reagents used in combination therapy did not exhibit an antitumor effect alone.

### [Introduction]

Immunotherapy by PD-1 blockade has brought a revolutionary impact on cancer treatment. This therapy is characterized by high efficacy against a wide variety of cancers, long-lasting antitumor effect and limited adverse effects^{9,10,11}. PD-1, which is expressed as a surface receptor in activated T cells, works as a suppressor in immune response. PD-1 binds to either one of its two ligands (PD-L1 and PD-L2) and phosphorylates the tyrosine residue in ITSM, resulting in the recruitment of tyrosine phosphatase SHP-2. Activation transmitting molecules such as Zap70 which have been phosphorylated by T cell receptor signals are dephosphorylated by SHP-2, resulting in inhibition of T cell activation^{12, 13}. Since PD-1 deficient mice developed autoimmune diseases in animal experiments, immunosuppressive function of PD-1 has been confirmed^{14,15,16}. Thus, PD-1 has been shown to work as an immune checkpoint and induces self-tolerance.

The present inventors have elucidated the basic role of PD-1 and revealed that immune responses to cancer or infection could be enhanced by modulating the PD-1 function ^{17,18}. Indeed, since the results of a clinical trial on melanoma were published in 2010¹⁹, clinical trials of the PD-1 blockade therapy on various cancers have been carried out and most of them have shown surprising effects. Currently, the PD-1 blockade therapy against melanoma, non-small-cell lung cancer and renal cell carcinoma is covered by medical insurance in Japan. The PD-1 blockade therapy has dramatically improved the survival rate of cancer patients, compared to not only conventional immunotherapy but also actually employed standard chemotherapy. Unfortunately, however, about 30-50% of patients are still non-responsive to the PD-1 blockade therapy. In order to deal with these non-responsive cases, the PD-1 blockade therapy has been combined with other immune checkpoint factors Lag3 and Tim3, cancer vaccines, radiotherapy, low-dose chemotherapy, etc²¹. However, a significant synergistic effect by combination therapies has not been reported yet.

Although the present inventors established the PD-1 blockade therapy in an animal model in 2002¹⁷, mechanisms for the tumoricidal effect and the breakage of immune tolerance by the PD-1 blockade therapy have not been elucidated yet. For example, checkpoint blockade immunotherapy mainly targets tumor-specific mutant antigens^{22,23}, but it is unknown whether effector T cells are activated at the tumor site or in draining lymph nodes. Moreover, when tumor is eradicated by PD-1 antibody therapy, it is even unknown what signal allows effector T cells to traffic to tumor site. It remains still unknown how to discriminate tumor-reactive cytotoxic T lymphocytes from tumor-non-reactive T lymphocytes, and how PD-1 signal blockade affects the activation/differentiation states of TR CTLs. Further, it remains unclear why the immune surveillance activated by PD-1 signal blockade in cancer patients lasts for years^{24,25}.

In order to establish a novel therapeutic strategy for making the PD-1 blockade therapy more effective, some of the above-listed questions must be answered. To this end, the present inventors analyzed TR CTLs focusing on metabolic reprogramming necessary for effector function^{1,2,3,26,27}. Interestingly, TR CTLs extracted from draining lymph nodes (DLNs) under the PD-1 blockade therapy showed larger mitochondrial mass, higher membrane potential, and high mitochondrial reactive oxygen species (ROS). These results show that PD-1 signal blockade increases mitochondrial activities, which is consistent with previous reports of *in vitro* studies^{28,29}.

Based on these results, the present inventors have hypothesized that stimulation of mitochondrial function in effector T cells induces enhancement of antitumor effect in the PD-1 blockade therapy. Indeed, activators of energy metabolic sensors AMP-activated Protein Kinase (AMPK), SIRT1 and Mechanistic Target of Rapamycin (mTOR) showed synergistic antitumor effects with the PD-1 blockade therapy. These findings may pave the way for developing combinatorial cancer therapies for those patients who are less responsive to the PD-1 blockade therapy.

### [Results]

### Augmentation of Mitochondrial Activities of Tumor-Reactive CD8⁺ T Cells in DLNs by PD-1 Blockade

When T cells are activated, factors necessary for rapid cell proliferation must be supplied promptly. The present inventors have examined the PD-1 blockade induced metabolic changes of tumor-reactive CD8⁺ T cells in DLNs. One of the issuts for the mechanistic analysis of the PD-1 blockade therapy is that comprehensive identification of TR CTLs is difficult since CTLs having a diverse T cell receptor repertoire responding to various tumor antigens are generated by PD-1 blockade ^{30,31}. In order to solve this problem, CellTrace-labeled CD45.1⁺ CD8 T cells were transplanted into CD45.2⁺ CD8 KO mice. Subsequently, the present inventors examined the proliferation of the labeled T cells in draining lymph nodes, assuming that tumor-reactive CD8⁺ T cells would proliferate selectively and vigorously (Fig. 2a). In MC38-bearing mice, the transplanted CD45.1⁺ CD8⁺T cells were divided into two populations: a vigorously dividing cell population (High Proliferation) and a less dividing cell population (Low Proliferation) (Fig. 2b). The absolute number and frequency of CD45.1⁺ CD8⁺T cells of High Proliferation in MC38-bearing mice were increased in the group treated with PD-L1 antibody compared to the group treated with control antibody IgG (Fig. 2b). Notably, PD-L1 antibody did not induce proliferation in non-tumor-bearing mice. These results strongly indicated that the vigorously dividing CD8⁺ T cells were indeed activated by tumor antigens (Fig.2b). Moreover, among CD45.1⁺ CD8⁺ T cells of DLNs mLama4 peptide (a mutated epitope of MC38)/MHC tetramer positive cell population was detected in a fraction of highly proliferating population induced by PD-1 blockade (Fig. 2c)²². Therefore, it is highly likely that the vigorously proliferating cell population in DLNs should be rich in TR CTLs.

Next, the present inventors examined the metabolic function of TR CTLs vigorously proliferating in DLNs. Since mitochondrial metabolism is considered to be important not only for T cell activation but also for continuous T cell proliferation or formation of memory T cells, the present inventors focused on the function of mitochondria in TR CTLs^{26,32}. Compared to less dividing cells, highly proliferating CD8⁺ T cells had larger mitochondrial mass, higher mitochondrial membrane potential and higher production of mitochondrial reactive oxygen species (ROS)³³. These results clearly show that mitochondria are activated in TR CTLs by PD-1 blockade (Fig. 2d). In agreement with these results, the oxygen consumption rate (OCR), an indicator of mitochondrial respiration, and basic consumption of ATP were remarkably higher in CD8⁺ T cells in DLNs of PD-L1 antibody-treated mice (Fig. 2e-f)³⁴. These results demonstrated that the mitochondrial metabolic rate increases with the proliferation of TR CTLs during the PD-1 blockade therapy.

### ROS Is Necessary for Enhancing the Antitumor Activity By PD-1 Blockade.

As described above, ROS increases remarkably as a result of PD-1 antibody therapy. ROS is generated in complexes I, II and III of the mitochondrial electron transport chain (ETC)³⁵. And mitochondrial ROS also functions as a signal transduction factor for expanding the antigenicity of T cells^{26, 32}. On the other hand, exogenous ROS or its generators, which are known to directly damage tumor cells (27), have been considered as candidates for cancer therapeutics³⁶. In view of these two aspects, the present inventors first tested whether a ROS generator alone would exhibit tumoricidal activity. When a ROS precursor, tert-butyl hydroperoxide solution (Luperox), was injected into MC38-bearing mice, no antitumor activity was observed (Fig. 3a). The present inventors further confirmed that there were no significant changes in immune-regulatory surface markers and transcriptional profiles of tumor cells treated with Luperox alone *in vivo* (Fig. 12). However, when combined with PD-L1 antibody, Luperox remarkably enhanced the antitumor effect and improved the survival rate of tumor-bearing mice (Fig. 3b, c). These data suggest that Luperox synergized with the antitumor effect of PD-L1 antibody, probably through activation of T-cells but not through a direct effect on tumor cells. A direct mitochondrial ROS generator paraquat also enhanced the effect for PD-1 blockade (Fig. 13a, b). As controls, oligomycin (a drug which destabilizes mitochondria to thereby inhibit ATP synthesis), carbonylcyanide-p-trifluoromethoxyphenylhydrazone (FCCP) and 2,4-dinitrophenol (DNP) were admdinistered³⁷. Unexpectedly, FCCP and DNP (which are uncouplers for mitochondria) augmented the efficacy of PD-L1 antibody therapy, and remarkably improved the survival rate of the mice compared to the group treated with PD-L1 antibody alone (Fig. 4a). Importantly, like Leuperox, both FCCP and DNP failed to exhibit any antitumor effect when administered alone. It was revealed that they would enhance the antitumor effect of PD-L1 antibody without having a direct effect on tumor cells (Fig. 4b). Furthermore, the phenotypic analysis and transcription profile analysis of tumor cells harvested from mice treated with FCCP alone did not show a significant difference, indicating again that uncouplers do not kill tumor cells directly but augment the function of tumoricidal killer T cells (Fig. 12). Since it was believed that mitochondrial uncoupling reduces the membrane potential and thus has a protective role from oxidative damage by reduction of ROS, the synergism of FCCP and DNP in antitumor effect was quite unexpected³⁷. About the uncouplers, there are contradictory reports: they either reduce or increase mitochondrial ROS production^{38,39}. The present inventors confirmed that the effect of combination therapy using PD-L1 antibody and uncouplers was inhibited by administration of MnTBAP (a synthetic metalloporphyrin acting as a ROS scavenger) (Fig. 4c). These results have revealed that the synergistic effect of the uncouplers is mediated by ROS signals. Curiously, the triple combination of PD-L1 antibody, DNP and Luperox showed a stronger antitumor activity (Fig. 5), suggesting that uncouplers may have other pathways unrelated with ROS signaling to augment the effect of PD-1 blockade.

### Uncouplers Promote the Differentiation of CD62L⁻ CD44⁺ Effector T Cells in DLNs and Augment Their Accumulation at the Tumor Site.

Then, how do uncouplers enhance the antitumor immunity of PD-L1 antibody through ROS signaling? In order to answer this question, the present inventors first examined the fraction of CD8⁺ T cell in both draining lymph nodes and tumor sites. As shown in Fig. 6a, the present inventors found that the absolute number and proportion of effector CD8⁺ T cells (CD62L⁻ CD44⁺ gated as P3) in DLNs significantly increased in the combination therapy group treated with PD-L1 antibody and FCCP compared with the group treated with anti-PD-L1 antibody alone. In contrast, the numbers of naive T cells (CD62L⁺ CD44⁻ gated as P1) and central memory T cells (CD62L⁺ CD44⁺ gated as P2) increased upon administration of PD-L1 antibody alone, but no further increase even in the addition of FCCP (Fig. 6a). It is of importance that the P3 population in any of the treatment groups contained larger mitochondrial mass, higher membrane potential and more ROS per cell than either the P1 or P2 population, and that membrane potential and ROS were significantly augmented when FCCP was combined with PD-L1 antibody (Fig. 6b). These observations suggest that (a) the dose of FCCP used did not have toxic effects such as decrease in mitochondrial mass or fall in membrane potential (which are expected at higher doses); (b) since high mitochondrial ROS production is observed in increased P3 cell population, synergistic effect by uncouplers is mediated by ROS; and (c) the feedback mechanism of mild mitochondrial damage may rather augment the mitochondrial activity (Fig. 6b).

Notably, P3 cell population even in CD8⁺ T cells infiltrating to the tumor significantly increased in accordance with such changes in DLNs (Fig. 6c). These data revealed that the combination therapy using PD-L1 antibody and uncouplers boosts the population size and the function of effector CD8⁺ T cells both in DLNs and at their target tumor site.

### Energy Metabolism Sensors AMPK and mTOR Are Involved in the Immune-Enhancing Effect of Uncouplers.

AMPK and mTOR are opposing energy metabolism sensors and the balance of phosphorylated AMPK and mTOR is believed to regulate the differentiation of CD8⁺ T cells^{1, 40,41,42,43,44}. Since it is known that increase of AMP/ATP ratio by uncouplers activates AMPK⁴⁵, the present inventors examined the state of phosphorylation of AMPK and mTOR in CD8⁺ T cells in DLNs of tumor-bearing mice treated by combination therapy using PD-L1 antibody and uncouplers. AMPK and its associated proteins ACC and SIRT1 were found to be activated at multiple time points after the combination therapy in CD8⁺ T cells harvested from mice treated with PD-L1 antibody and DNP or FCCP (Fig.7a). Unexpectedly, however, mTOR and its associated proteins S6K and 4EBP1 were also found to be activated after the combination therapy (Fig. 7a).

However, these puzzling results can be explained by the presence of heterogeneous cell populations, each carrying distinct AMPK/mTOR balance, within the CD8⁺ T cells in DLNs. Indeed, more p-AMPK was activated than p-mTOR in the P2 population, whereas the P3 population expressed more p-mTOR than p-AMPK (Fig. 8). Based on these results, the present inventors next examined which activation of the two energy metabolism sensors, mTOR or AMPK, directly enhances the efficacy of the PD-1 blockade therapy. As shown in Fig. 7b, both the mTOR activator and the AMPK activator moderately augmented the efficacy of the PD-1 blockade therapy in the early phase (before day 20), whereas the triple combination of mTOR activator, AMPK activator and PD-L1 antibody further enhanced the effect of PD-L1 antibody and improved animal survival. These results indicate that activation of both mTOR and AMPK is involved in the synergistic tumoricidal activity of PD-L1 antibody and the uncouplers.

Since the combined therapy using PD-L1 antibody and uncoupler(s) increased SIRT1 (Fig. 7a), the present inventors examined whether the activation of SIRT1 (an NAD-dependent protein deacetylase) would affect the efficacy of PD-L1 antibody therapy. It has been reported that SIRT1 is activated by Resveratrol, a polyphenol reportedly having a potential tumor inhibitory effects ^{6,46}. Further, it has been reported that a low dose of Resveratrol causes activation of AMPK and biogenesis of mitochondria in an SIRT1-dependent manner⁶, and that Resveratrol also assists the mTOR signaling pathway under specific conditions^{47,48,49}. Indeed, combination therapy using PD-L1 antibody and Resveratrol remarkably reduced tumor sizes and improved survival rate, compared to monotherapy with PD-L1 antibody (Fig. 7c). These results indicate that combination of PD-L1 antibody with activation of energy metabolism sensors AMPK and mTOR, as well as SIRT1 augments the proliferation and function of TR CTLs *in vivo.*

### PGC-1α Activators Enhance the Therapeutic Effect of PD-1 Antibody.

PGC-1α, a transcriptional cofactor regulated by either AMPK or mTOR, is known to enhance mitochondrial biogenesis and oxidative phosphorylation^{45,46}. The present inventors found that combination therapy using FCCP, mTOR activator or AMPK activator together with PD-L1 antibody increased the expression levels of PGC-1α protein and mRNA, a result in agreement with previous reports (Fig. 11a)^{37,45,46}. When PD-L1 antibody alone was administered, expression of PGC-1α protein was increased but expression of PGC-1α mRNA was reduced. This result is probably due to the involvement of multiple steps (transcription, translation, protein stabilization, etc.) in PGC-1α regulation^{46,47}. It is reported that PGC-1α functions through correlating with transcription factors such as NRFs and PPARs⁴⁶. The present inventors tested whether oltipraz, an activator of PGC-1α/NRF2, and bezafibrate, an activator of PGC-1α/PPARs, would enhance the antitumor effect of PD-L1 antibody^{48,29}. As a result, both oltipraz and bezafibrate enhanced the tumor growth inhibitory effect of PD-L1 antibody and improved the survival rate of tumor-bearing mice (Fig. 11b).

To examine whether this PD-1 antibody combination therapy is applicable to tumors other than MC38, the present inventors transplanted MethA, a murine skin sarcoma cell line, into BALB/c mice intradermally and tested the combination therapy using FCCP, paraquat or oltipraz. As a result, all of the chemicals tested enhanced the antitumor effect of PD-L1 antibody (Fig. 14). This indicates that the combination therapy using mitochondrial activators together with PD-1 inhibitory antibody is applicable to various tumors of different genetic backgrounds.

### Combination Therapy with FCCP or Bezafibrate Enhances T-bet Expression on CTLs.

T-bet, a critical transcription factor for cytokine secretion and activation of TR CTLs by PD-1 blockade, is known to be activated by mTOR through FOXO1 inhibition. The present inventors examined how combination therapy of FCCP and PD-L1 antibody affects T-bet and Eomes expression. In agreement with the above-described finding that combination therapy using PD-L1 antibody and FCCP activated mTOR, FCCP increased T-bet expression but not Eomes expression in CD8⁺ cells (Fig. 15a). Interestingly, in agreement with a report that FOXO1 regulates T-bet and Eomes in opposite directions, bezafibrate (which activates mTOR's downstream transcription factors) also increased T-bet but reduced Eomes instead (Fig. 15a). Further, IFN-γ production, which is one of killer T cell's functions, was enhanced in tumors under combination therapy (Fig. 15b). This coincides with the enhancemeced T-bet expression which is critical to Th1-type immunity. These results suggest that bezafibrate probably activates mitochondria and, after the occurrence of a positive feedback, eventually activates mTOR. Taken together, the molecular mechanism of the synergism of uncouplers and PD-1 blockade is mediated by activation of AMPK, mTOR and their downstream transcription factors including NRF2, PPARs and coupling factors such as PGC-1α (Fig. 1).

### [Discussion]

The present inventors have demonstrated that PD-L1 antibody treatment activates mitochondria with higher ROS in TR CTLs, and that administration of ROS generators enhances the antitumor effect of PD-1 blockade. Unexpectedly, it has been found that the two well-known mitochondrial uncouplers, FCCP and DNP, also enhance the antitumor effect of PD-L1 antibody. Since ROS scavengers cancelled the synergistic effect of the uncouplers, it is clear that the synergistic effect of the uncouplers is dependent on ROS generation. The present results were quite unexpected because mitochondrial uncouplers was considered to have an effect for suppressing ROS production and might be useful for treating oxidation stress-related diseases such as ischemic damage, cardiac failure, insulin resistance, obesity, aging, etc.⁵⁰ Administration of the uncouplers alone did not show any effect on the proliferation of T cells or tumor growth. Therefore, the uncouplers at the minimum dose used in the present study were found to be rather effective for supplementing the immunological events or immune reactions induced by PD-1 blockade.

Resveratrol, a natural polyphenol, is known for its cardiovascular improvement, antiaging effect and antitumor effect. It implies that Resveratrol induces mitochondrial biogenesis and functional metabolic change to protect hosts from diseases. The present inventors revealed that Resveratrol at lower doses enhances the efficacy of the PD-1 blockade therapy. Like uncouplers, Resveratrol itself was not involved in tumor growth or, if anything, increased tumor. However, when used in combination with PD-L1 antibody, Resveratrol exhibited a remarkable tumor inhibitory effect and improved the survival rate in animal models.

How do these chemicals cause synergistic effects with the PD-1 blockade therapy in similar manners? Mitochondrial energy metabolism is closely linked with intracellular metabolism via mTOR and AMPK^{5,6,49,51}. The present inventors confirmed that AMPK and associated proteins thereof are activated by combination therapy using PD-L1 antibody and uncouplers. Importantly, all of the direct activators of AMPK or SIRT1 enhanced the antitumor effect of PD-L1 antibody.

The present inventors demonstrated that combination therapy using uncouplers and PD-1 blockade activates not only AMPK pathway but also mTOR pathway and that direct activators of mTOR also enhance the effect of PD-L1 antibody. These results were unexpected because previous reports have suggested that activation of mTOR pathway competes with phosphorylation of AMPK^{1,41,42,43,52}. However, this observations may be explicable without contradiction with previous reports, considering that the isolated CD8⁺ T cells consist of heterogeneous cells being at different stages of activation and functions, and also given biochemical reactions and dynamic changes. The present inventors speculate that activation of mTOR pathway leads to the expression of T-bet in DLN CTLs stimulated by FCCP or bezafibrate together with PD-1 blockade. T-bet is important for the antitumor function of CTLs as well as for the differentiation of memory precursors which potentially supply terminally differentiated effector CTLs required for tumor regression^{55,56}. On the other hand, terminally differentiated CTLs which strongly express EOMES are reported to be in an immunologically tolerized state by chronic antigen recognition^{56,57}. According to the experimental results obtained by the present inventors, the combination of PD-L1 antibody therapy with FCCP or bezafibrate increased T-bet and IFN-γ expression but rather decreased EOMES expression. This is a result not contradictory with our conclusion that the combination therapy expands effector-memory cell population (P3 population) with activated mitochondria.

PGC-1α is a molecule which regulates a series of transcription factors involved in mitochondrial biogenesis and mitochondrial oxidative phosphorylation. Activation of AMPK or mTOR augments PGC-1α expression and activates PGC-1α through phosphorylation. The present inventors have demonstrated that the PD-1 blockade combination therapy with uncouplers increases PGC-1α expression and that PGC-1α activators (bezafibrate and oltipraz) also synergize with PD-L1 antibody in tumor treatment models. These results clearly revealed that PGC-1α is a key molecule that triggers mitochondrial activation and expansion and induces the feed-forward signaling cycle of mTOR and AMPK activation pathways. In addition, uncouplers and bezafibrate, when combined with PD-L1 antibody, activated T-bet, a critical cytokine regulator located downstream of mTOR.

The whole image of the current hypothesis concerning the flow of mechanisms described so far is summarized in Fig. 1.
a) PD-1 blockade induces mitochondrial expansion and proliferation in activated CD8⁺ T cells.
b) Uncouplers and ROS generators further increase mitochondrial mass, resulting in increased ROS, which probably activates AMPK and mTOR via unknown pathways³⁶.
c) Activation of AMPK and mTOR increases PGC-1α expression and activates PGC-1α.
d) Finally, PGC-1α and its cofactors, NRFs and PPARs, increase the expression of a series of transcription factors which activate oxidation and oxidative phosphorylation of fatty acids, and they also induce mitochondrial expansion which promotes activation and differentiation ofCTLs.

Very recently, it was reported that PD-1 signal pathway inhibits mitochondrial activation and PGC-1α expression, which also do not contradict the hypothesis of the present inventors^{63,64}. All chemicals used in this study, namely ROS, uncouplers, AMPK activators, mTOR activators and PGC-1α activators, led to CD8⁺ T cell activation and differentiation through mitochondrial activation and expansion when combined with PD-1 antibody, whereas those chemicals exhibited no such effect when administered alone.

The current results demonstrate the sufficient efficacy of the revolutionary combination therapy applicable to those cancer patients who do not respond efficiently to PD-1 antibody therapy. Since mitochondrial activation did not occur in CTLs derived from mice bearing PD-1 blockade-insensitive tumors, the present inventors' study could also lead to research of predictive biomarkers for the efficacy of the PD-1 antibody therapy. It would be important to examine whether the quantities of serum metabolites changing in response to mitochondrial metabolic alterations, the OCR activity in CD8⁺ T cells in samples collected before and after PD-1 antibody therapy, and/or RNA expression associated with mitochondrial activation signals could serve as biomarkers for responsivity to the PD-1 antibody therapy. Moreover, synergistic enhancement of the PD-1 antibody therapy by chemicals will help reduce the amounts of expensive PD-1 antibodies which have been discussed as endangering the social security system.

Currently, cancer treatment by PD-1/PD-L1 blockade have two big problems. The first problem is that the PD-1 blockade therapy is not effective on all patients, as already described above. Therefore, clinically applicable chemicals which could enhance the antitumor effect of the PD-1 blockade therapy have been developed. The current results would provide alternatives of new combination therapies to unresponsive patients for the PD-1 blockade therapy. The second problem is about adverse effects of the PD-1 blockade therapy alone or combination therapy, taking examples of toxicities peculiar to chemicals and autoimmune diseases by excessive immune responses⁵⁷. Such therapeutic interventions to improve the immunotherapy may well accompany increased adverse effects. Although no particularly pronounced autoimmune reactions were observed in the combination therapies using PD-L1 antibody and the chemicals in this study, considering the clinical applications, this risk must be evaluated using the autoimmune disease mouse model previously established by the present inventors ^{14,15,16}. DNP was used in 1930's as a diet supplement or a drug increasing metabolic rate ⁵⁸. However, due to its effect of decay of proton-motive force, DNP caused severe health problems and its use has been banned⁵⁹. Although FCCP has been used broadly in studying mitochondrial bioenergy, this chemical is yet to be clinically applied because it has cytotoxicity and a certain degree of plasma membrane depolarization effect⁵⁰. In the current study, the present inventors have demonstrated that the five chemicals enhance the antitumor effect of PD-1 blockade. Among them, a natural polyphenol Resveratrol, which is an SIRT1 activator, is ideal^{5, 51}, considering that this chemical is frequently tested in treating metabolic diseases and taken daily as a supplement. Although the antitumor effect of Resveratrol alone has been reported, it is still controversial due to certain reasons including its dose dependency⁶⁰. The dose used in the current study was at minimum level, and as far as the present inventors have tested, no tumor growth inhibitory effect was observed (rather, reverse was the case). The other chemicals which the present inventors identified for use in combination therapy were uncouplers and activators for downstream signaling pathways of ROS. Considering reduction of off-targets, those therapies targeting the downstream signaling pathways of mTOR, AMPK or SIRT1 will potentially enhance the antitumor effect and yet mitigate undesirable adverse effects.

In conclusion, the present inventors have identified those chemicals which enhance the efficacy of the PD-1 blockade therapy by regulating mitochondrial energy metabolism checkpoints. These findings open a new avenue for the study of PD-1-mediated T-cell regulation as well as a combinatorial treatment strategy for cancer patients unresponsive to the PD-1 blockade therapy and/or for patients with infectious diseases. Notably, oltipraz and bezafibrate have already been used as clinical medicines and, therefore, they are applicable to clinical studies of combination therapy with PD-1 antibody.

### [Procedures and Materials]

### Mice and Cells

All mice were maintained under specific pathogen-free (SPF) conditions at the Institute of Laboratory Animals, Graduate School of Medicine, Kyoto University and used under appropriate protocols. C57BL/6 and BALB/c mice (5 to 6 wk old) were obtained from Charles River Laboratories Japan (Yokohama). Murine colon adenocarcinoma MC38 cells were kindly provided by Dr. Jim Allison (Memorial Sloan-Kettering Cancer Center, New York, NY). Fibrosarcoma MethA cells were obtained from the Cell Resource Center for Biomedical Research (Sendai, Japan). These cell lines were cultured in DMEM (Invitrogen) with 10% heat inactivated FBS and 1% antifungal antibiotic (Invitrogen). These cell lines are not infected with mycobacteria.

### Mouse Therapy Model

MC38 (5 × 10⁵) and MethA (5 × 10⁵) cells were intradermally injected on the right flank. MC38 was inoculated into C57BL/6 mice and MethA was inoculated into BALB/c mice. For monotherapy, mice were intraperitoneally injected with 150 µg of PD-L1 mouse antibody (1-111A) (prepared by the laboratory of the present inventors) 5 days after the tumor inoculation. Therapy with PD-L1 antibody alone was repeated three times every 5 days. For combination therapy, administration of PD-L1 antibody and chemical agents started 7 days after tumor injection. Chemical agents were injected at a regular interval of 2 days and PD-L1 antibody at a regular interval of 5 days (shown in Fig. 3b). Tumors were measured on every alternate day, and tumor volumes were calculated using the formula for typical ellipsoid, π × (length ×breadth × height/6).

### CD8 Deficient Mouse Model

In the CD8 deficient mouse model, CD8⁺ T cells were isolated from CD45.1⁺ mice using an autoMACS Pro Separator (Miltenyi Biotec). Isolated CD45.1⁺ CD8⁺ T cells were labeled with CellTrace Violet (Thermo Fisher Scientific) by incubating cells for 20 min with the CellTrace diluted in PBS. After washing, cells were injected into the tail vein of CD45.2⁺ CD8^{-/-} mice. After 5 days, MC38 (5 × 10⁵ cells) were intradermally injected, and PD-L1 antibody was intraperitoneally administered 8 days after the tumor inoculation.

### Chemical Reagents

The following chemical reagents were used at the indicated concentrations for combination therapy. AMPK activator: A769662, 6 mg/kg (Abcam); mTOR activator: MHY1485, 3 µg/kg (Sigma-Aldrich); uncoupler: carbonylcyanide-p-trifluoromethoxyphenylhydrazone (FCCP), 1.2 mg/kg (Abcam); uncoupler: 2,4-Dinitrophenol (DNP), 1.7 mg/kg (Aldrich); SIRT1 activator: resveratrol, 0.5 mg/kg (Abcam); ROS scavenger: Mn (III) tetrakis (4-benzoic acid) porphyrin (MnTBAP), 1.25 mg/kg (Calbiochem); ROS generator: tert-butyl hydroperoxide solution (Luperox TBH70X), 200 µL/kg (Sigma-Aldrich); ATP synthase inhibitor: oligomycin, 250 µg/kg (Sigma-Aldrich); paraquat 2 mg/kg (Nakalai Tesque); oltipraz 1.9 mg/kg (Sigma); bezafibrate 1 mg/kg (ChemCruz).

All reagents were freshly prepared just before use. Each reagent was dissolved in the solvent indicated in the instructions. Note that the AMPK activator, mTOR activator, uncouplers and SIRT1 activator were prepared from fresh unused vials in each series of experiments. Dissolved reagents were diluted in PBS, and a 200 µl aliquot was injected per mouse.

### Cytokine Bead Array Assay (CBA)

Concentrations of various cytokines in sera harvested from treated mice were quantitatively measured using beads coated with antibodies against the different mouse cytokines IL-17A, IL-21, MIG, TNF-α, IFN-γ, IL-1β, IL-2, IL-4, IL-6, IL-10 and IL-12. Stained samples were run on a FACS Canto II flow cytometer (BD Biosciences) following the instructions. The data were analyzed using FACS Array software v3.0 (BD Biosciences).

### Enzyme-Linked Immunoassay (ELISA)

MC38 was inoculated into mice and treated with PD-L1 antibody, as described for monotherapy using PD-L1 antibody alone. MIG levels in sera were measured quantitatively with an MIG ELISA Kit (Abcam) following the instructions in the kit.

### Cell Preparation

For analyzing draining lymph nodes, cells from axillary, brachial, and inguinal lymph nodes on the right side of tumor-inoculated mice were harvested and mixed. Averaged cell numbers per one lymph node were used as absolute cell numbers. For tumor analysis, tumor tissues were minced into 2- to 3-mm pieces with scissors and digested with collagenase type IV (Thermo Fisher Scientific) using a gentle MACS Dissociator (Miltenyi Biotec). The numbers of tumor cells per mg were used as absolute numbers. Tumor cells were isolated from digested tumor tissues using a Tumor Cell Isolation Kit, mouse (Miltenyi Biotec). This kit purifies tumor cells by excluding lymphocytes, red blood cells, fibroblasts, endothelial cells and tumor-associated stromal cells. The resultant tumor cell population was further isolated/purified with FACS Aria (BD Biosciences).

### Flow Cytometry Analysis

The following antibodies recognizing the indicated antigens were used: CD44 (1M7), CD45.2 (104), CD45.1 (A20), CD8 (53-6.7), CD62L (MEL-14), T-bet (4B/O) and IFN-γ (XMG1.2), MHC class I (AF6-88.5), CD155 (Tx56) and VISTA (MIH63) from BioLegend; p-AMPK (EPR5683) from Ancam; p-mTOR (MRRBY), Eomes (Danllmag) and PD-L1 (M1H5) from eBioscience. All flow cytometry experiments were performed on a FACS Canto II (BD Biosciences) and analyzed using FlowJo software (FLOWJO, LLC). For assessment of intracellular phosphoproteins, cells were permeabilized with 0.5% Triton-X and fixed with 1.5% PFA before staining. Determination of mitochondrial mass, membrane potential, mitochondrial superoxide and cellular ROS was performed using MitoTracker Green, Mito-Tracker Deep Red, MitoSOX Red, and CellROX Green reagents, respectively (all from Life Technologies). These dyes were added to cells to a final concentration of 0.125 µM, 0.125 µM, 5.0 µM and 0.625 µM, respectively, and incubated at 37 °C in a 5% CO₂ humidified incubator for 30 min.

### Measurement of Oxygen Consumption Rates

Oxygen consumption rates were measured using an XF96 Extracellular Flux Analyzer (Seahorse Bioscience). CD8⁺ T cells (4 × 10⁵) per well were seeded in a determined XF96 plate. The four chemical modulators of mitochondrial oxidative phosphorylation, which came with an XF Cell Mito Stress Test Kit (Seahorse Bioscience), were added to the module sequentially. In brief, basal OCR measurement was performed after sequential addition of oligomycin, FCCP, and rotenone/antimycin A. ATP turnover was defined as (last rate measured before oligomycin addition) - (minimum rate measured after oligomycin addition)³⁴.

### Real-Time RT-PCR

RNA was isolated from CD8⁺ T cells or tumor cells with the RNeasy Mini Kit (Qiagen), followed by cDNA synthesis by reverse transcription. Real-time PCR for PGC-1α was performed using the following primers: Forward ACTCGGATTGCTCCGGCCCT (SEQ ID NO: 1) and Reverse ACTGACGGCCTAACTCCACCCA (SEQ ID NO: 2). To analyze the expression levels of genes associated with apoptosis and mitochondrial energy metabolism, RT2 Profiler PCR Array Gene Expression Kit PAMM-012Z or PAMM-008Z (Qiagen) was used. The list of genes included in this assay can be found in http://www.sabiosciences.com/Apoptosis.php

### Western Blotting

CD8⁺ T cells were isolated using mouse CD8 Microbeads (Miltenyi Biotec). After washing with PBS twice, 2 × 10⁶ cells were solubilized in a lysis buffer containing 30 mM Tris-HCl (pH.7.4), 150 mM NaCl, 10% glycerol, 0.1% SDS, 1% Triton-X-100, 0.05% Na-Doc, 5 mM EDTA (pH.8.0), protease inhibitor mixture (Roche Molecular Biochemicals) and phosphatase inhibitors (Nacalai Tesque). After measurement of protein concentrations by DC protein assay (Bio-Rad), 4 µg of proteins was mounted on 4 to 20% gradient Mini-PROTEAN TGX Gels (Bio-Rad) and electroblotted onto nitrocellulose membranes, which were then incubated in a blocking buffer of TBS containing 1% BSA. Primary antibody incubations were carried out overnight at 4 °C in blocking buffer. After washing, secondary antibody incubations were carried out at room temperature for 40 min in blocking buffer. Blots were developed with enhanced chemiluminescence (Amersham Pharmacia). Primary antibodies recognizing the following proteins were used: Phospho-mTOR (P-mTOR) (Cat#5536), Phospho-AMPKα (P-AMPK) (#2535), Phospho-p70 S6 Kinase (P-S6K) (#9205), 4E-BP1 (#9644), Phospho-Acetyl-CoA Carboxylase (P-ACC) (#3661) and SIRT1 (#9475). All the primary antibodies were obtained from Cell Signaling Technology. An antibody recognizing PGC-1α (SC-13067) was obtained from Santa Cruz.

### Statistical Analysis

Statistical analysis was performed using Prism 6. One-way ANOVA analysis (one-way analysis of variance) followed by Sidak's multiple comparison test was used to analyze three or more variables. For comparison of two groups, Student t test was used. All statistical analyses were two-sided assuming parametric data. P value of <0.05 was considered significant. The variations of data were evaluated as the means ± SEM. Five or more samples are believed to be appropriate for the sample size estimate in the current study. Samples and animals were randomly chosen from the pool and treated. No blinding test was used for the treatment of samples and animals.

### REFERENCES

1. Chi H. Regulation and function of mTOR signalling in T cell fate decisions. Nat Rev Immunol 2012, 12(5): 325-338.
2. Cunningham JT, Rodgers JT, Arlow DH, Vazquez F, Mootha VK, Puigserver P. mTOR controls mitochondrial oxidative function through a YY1-PGC-1 alpha transcriptional complex. Nature 2007, 450(7170): 736-740.
3. Toyama EQ, Herzig S, Courchet J, Lewis TL, Jr., Loson OC, Hellberg K, et al. Metabolism. AMP-activated protein kinase mediates mitochondrial fission in response to energy stress. Science 2016, 351(6270): 275-281.
4. Ventura-Clapier R, Garnier A, Veksler V. Transcriptional control of mitochondrial biogenesis: the central role of PGC-1alpha. Cardiovasc Res 2008, 79(2): 208-217.
5. Canto C, Auwerx J. Targeting sirtuin 1 to improve metabolism: all you need is NAD(+)? Pharmacol Rev 2012, 64(1): 166-187.
6. Price NL, Gomes AP, Ling AJ, Duarte FV, Martin-Montalvo A, North BJ, et al. SIRT1 is required for AMPK activation and the beneficial effects of resveratrol on mitochondrial function. Cell metabolism 2012, 15(5): 675-690.
7. Scarpulla RC. Metabolic control of mitochondrial biogenesis through the PGC-1 family regulatory network. Biochim Biophys Acta 2011, 1813(7): 1269-1278.
8. Blaschke F, Takata Y, Caglayan E, Law RE, Hsueh WA. Obesity, peroxisome proliferator-activated receptor, and atherosclerosis in type 2 diabetes. Arterioscler Thromb Vasc Biol 2006, 26(1): 28-40.
9. Couzin-Frankel J. Breakthrough of the year 2013. Cancer immunotherapy. Science 2013, 342(6165): 1432-1433.
10. Topalian SL, Drake CG, Pardoll DM. Immune checkpoint blockade: a common denominator approach to cancer therapy. Cancer Cell 2015, 27(4): 450-461.
11. Okazaki T, Chikuma S, Iwai Y, Fagarasan S, Honjo T. A rheostat for immune responses: the unique properties of PD-1 and their advantages for clinical application. Nat Immunol 2013, 14(12): 1212-1218.
12. Okazaki T, Maeda A, Nishimura H, Kurosaki T, Honjo T. PD-1 immunoreceptor inhibits B cell receptor-mediated signaling by recruiting src homology 2-domain-containing tyrosine phosphatase 2 to phosphotyrosine. Proc Natl Acad Sci U S A 2001, 98(24): 13866-13871.
13. Chemnitz JM, Parry RV, Nichols KE, June CH, Riley JL. SHP-1 and SHP-2 associate with immunoreceptor tyrosine-based switch motif of programmed death 1 upon primary human T cell stimulation, but only receptor ligation prevents T cell activation. J Immunol 2004, 173(2): 945-954.
14. Nishimura H, Nose M, Hiai H, Minato N, Honjo T. Development of lupus-like autoimmune diseases by disruption of the PD-1 gene encoding an ITIM motif-carrying immunoreceptor. Immunity 1999, 11(2): 141-151.
15. Nishimura H, Okazaki T, Tanaka Y, Nakatani K, Hara M, Matsumori A, et al. Autoimmune dilated cardiomyopathy in PD-1 receptor-deficient mice. Science 2001, 291(5502): 319-322.
16. Okazaki T, Tanaka Y, Nishio R, Mitsuiye T, Mizoguchi A, Wang J, et al. Autoantibodies against cardiac troponin I are responsible for dilated cardiomyopathy in PD-1-deficient mice. Nat Med 2003, 9(12): 1477-1483.
17. Iwai Y, Ishida M, Tanaka Y, Okazaki T, Honjo T, Minato N. Involvement of PD-L1 on tumor cells in the escape from host immune system and tumor immunotherapy by PD-L1 blockade. Proc Natl Acad Sci U S A 2002, 99(19): 12293-12297.
18. Iwai Y, Terawaki S, Ikegawa M, Okazaki T, Honjo T. PD-1 inhibits antiviral immunity at the effector phase in the liver. J Exp Med 2003, 198(1): 39-50.
19. Brahmer JR, Drake CG, Wo liner I, Powderly JD, Picus J, Sharfman WH, et al. Phase I study of single-agent anti-programmed death-1 (MDX-1106) in refractory solid tumors: safety, clinical activity, pharmacodynamics, and immunologic correlates. J Clin Oncol 2010, 28(19): 3167-3175.
20. Zou W, Wolchok JD, Chen L. PD-L1 (B7-H1) and PD-1 pathway blockade for cancer therapy: Mechanisms, response biomarkers, and combinations. Sci Transl Med 2016, 8(328): 328rv324.
21. Mahoney KM, Rennert PD, Freeman GJ. Combination cancer immunotherapy and new immunomodulatory targets. Nat Rev Drug Discov 2015, 14(8): 561-584.
22. Gubin MM, Zhang X, Schuster H, Caron E, Ward JP, Noguchi T, et al. Checkpoint blockade cancer immunotherapy targets tumour-specific mutant antigens. Nature 2014, 515(7528): 577-581.
23. McGranahan N, Furness AJ, Rosenthal R, Ramskov S, Lyngaa R, Saini SK, et al. Clonal neoantigens elicit T cell immunoreactivity and sensitivity to immune checkpoint blockade. Science 2016, 351(6280): 1463-1469.
24. Topalian SL, Hodi FS, Brahmer JR, Gettinger SN, Smith DC, McDermott DF, et al. Safety, activity, and immune correlates of anti-PD-1 antibody in cancer. N Engl J Med 2012, 366(26): 2443-2454.
25. Brahmer JR, Tykodi SS, Chow LQ, Hwu WJ, Topalian SL, Hwu P, et al. Safety and activity of anti-PD-L1 antibody in patients with advanced cancer. N Engl J Med 2012, 366(26): 2455-2465.
26. Sena LA, Li S, Jairaman A, Prakriya M, Ezponda T, Hildeman DA, et al. Mitochondria are required for antigen-specific T cell activation through reactive oxygen species signaling. Immunity 2013, 38(2): 225-236.
27. O'Sullivan D, Pearce EL. Targeting T cell metabolism for therapy. Trends Immunol 2015, 36(2): 71-80.
28. Chang CH, Qiu J, O'Sullivan D, Buck MD, Noguchi T, Curtis JD, et al. Metabolic Competition in the Tumor Microenvironment Is a Driver of Cancer Progression. Cell 2015, 162(6): 1229-1241.
29. Patsoukis N, Bardhan K, Chatterjee P, Sari D, Liu B, Bell LN, et al. PD-1 alters T-cell metabolic reprogramming by inhibiting glycolysis and promoting lipolysis and fatty acid oxidation. Nat Commun 2015, 6: 6692.
30. Tumeh PC, Harview CL, Yearley JH, Shintaku IP, Taylor EJ, Robert L, et al. PD-1 blockade induces responses by inhibiting adaptive immune resistance. Nature 2014, 515(7528): 568-571.
31. Kvistborg P, Philips D, Kelderman S, Hageman L, Ottensmeier C, Joseph-Pietras D, et al. Anti-CTLA-4 therapy broadens the melanoma-reactive CD8+ T cell response. Sci Transl Med 2014, 6(254): 254ra128.
32. Weinberg SE, Sena LA, Chandel NS. Mitochondria in the regulation of innate and adaptive immunity. Immunity 2015, 42(3): 406-417.
33. Jang KJ, Mano H, Aoki K, Hayashi T, Muto A, Nambu Y, et al. Mitochondrial function provides instructive signals for activation-induced B-cell fates. Nat Commun 2015, 6: 6750.
34. van der Windt GJ, Chang CH, Pearce EL. Measuring Bioenergetics in T Cells Using a Seahorse Extracellular Flux Analyzer. Curr Protoc Immunol 2016, 113: 3 16B 11-13 16B 14.
35. Turrens JF. Mitochondrial formation of reactive oxygen species. J Physiol 2003, 552(Pt 2): 335-344.
36. Trachootham D, Alexandre J, Huang P. Targeting cancer cells by ROS-mediated mechanisms: a radical therapeutic approach? Nat Rev Drug Discov 2009, 8(7): 579-591.
37. Krauss S, Zhang CY, Lowell BB. The mitochondrial uncoupling-protein homologues. Nat Rev Mol Cell Biol 2005, 6(3): 248-261.
38. Olsson M, Wilson M, Uller T, Isaksson C. Free radicals run in lizard families without (and perhaps with) mitochondrial uncoupling. Biol Lett 2009, 5(3): 345-346.
39. Han YH, Kim SH, Kim SZ, Park WH. Carbonyl cyanide p-(trifluoromethoxy) phenylhydrazone (FCCP) as an O2(*-) generator induces apoptosis via the depletion of intracellular GSH contents in Calu-6 cells. Lung Cancer 2009, 63(2): 201-209.
40. Araki K, Ahmed R. AMPK: a metabolic switch for CD8+ T-cell memory. Eur J Immunol 2013, 43(4): 878-881.
41. Buck MD, O'Sullivan D, Pearce EL. T cell metabolism drives immunity. J Exp Med 2015, 212(9): 1345-1360.
42. Siska PJ, Rathmell JC. T cell metabolic fitness in antitumor immunity. Trends Immunol 2015, 36(4): 257-264.
43. Finlay D, Cantrell DA. Metabolism, migration and memory in cytotoxic T cells. Nat Rev Immunol 2011, 11(2): 109-117.
44. Blagih J, Coulombe F, Vincent EE, Dupuy F, Galicia-Vazquez G, Yurchenko E, et al. The energy sensor AMPK regulates T cell metabolic adaptation and effector responses in vivo. Immunity 2015, 42(1): 41-54.
45. Rohas LM, St-Pierre J, Uldry M, Jager S, Handschin C, Spiegelman BM. A fundamental system of cellular energy homeostasis regulated by PGC-1alpha. Proc Natl Acad Sci USA 2007, 104(19): 7933-7938.
46. Jang M, Cai L, Udeani GO, Slowing KV, Thomas CF, Beecher CW, et al. Cancer chemopreventive activity of resveratrol, a natural product derived from grapes. Science 1997, 275(5297): 218-220.
47. Zong Y, Sun L, Liu B, Deng YS, Zhan D, Chen YL, et al. Resveratrol inhibits LPS-induced MAPKs activation via activation of the phosphatidylinositol 3-kinase pathway in murine RAW 264.7 macrophage cells. PLoS One 2012, 7(8): e44107.
48. Leontieva OV, Paszkiewicz G, Demidenko ZN, Blagosklonny MV. Resveratrol potentiates rapamycin to prevent hyperinsulinemia and obesity in male mice on high fat diet. Cell Death Dis 2013, 4: e472.
49. Hong S, Zhao B, Lombard DB, Fingar DC, Inoki K. Cross-talk between sirtuin and mammalian target of rapamycin complex 1 (mTORC1) signaling in the regulation of S6 kinase 1 (S6K1) phosphorylation. J Biol Chem 2014, 289(19): 13132-13141.
50. Kenwood BM, Weaver JL, Bajwa A, Poon IK, Byrne FL, Murrow BA, et al. Identification of a novel mitochondrial uncoupler that does not depolarize the plasma membrane. Mol Metab 2014, 3(2): 114-123.
51. de Oliveira MR, Nabavi SF, Manayi A, Daglia M, Hajheydari Z, Nabavi SM. Resveratrol and the mitochondria: From triggering the intrinsic apoptotic pathway to inducing mitochondrial biogenesis, a mechanistic view. Biochim Biophys Acta 2016, 1860(4): 727-745.
52. Inoki K, Kim J, Guan KL. AMPK and mTOR in cellular energy homeostasis and drug targets. Annu Rev Pharmacol Toxicol 2012, 52: 381-400.
53. Araki K, Turner AP, Shaffer VO, Gangappa S, Keller SA, Bachmann MF, et al. mTOR regulates memory CD8 T-cell differentiation. Nature 2009, 460(7251): 108-112.
54. Pearce EL, Walsh MC, Cejas PJ, Harms GM, Shen H, Wang LS, et al. Enhancing CD8 T-cell memory by modulating fatty acid metabolism. Nature 2009, 460(7251): 103-107.
55. Rolf J, Zarrouk M, Finlay DK, Foretz M, Viollet B, Cantrell DA. AMPKalpha1: a glucose sensor that controls CD8 T-cell memory. Eur J Immunol 2013, 43(4): 889-896.
56. Eikawa S, Nishida M, Mizukami S, Yamazaki C, Nakayama E, Udono H. Immune-mediated antitumor effect by type 2 diabetes drug, metformin. Proc Natl Acad Sci U SA2015, 112(6): 1809-1814.
57. Eigentler TK, Hassel JC, Berking C, Aberle J, Bachmann O, Grunwald V, et al. Diagnosis, monitoring and management of immune-related adverse drug reactions of anti-PD-1 antibody therapy. Cancer Treat Rev 2016, 45: 7-18.
58. Koch RA, Lee RC, Tainter ML. Dinitrophenol on Liver Function. Cal West Med 1935, 43(5): 337-339.
59. Grundlingh J, Dargan PI, El-Zanfaly M, Wood DM. 2,4-dinitrophenol (DNP): a weight loss agent with significant acute toxicity and risk of death. J Med Toxicol 2011, 7(3): 205-212.
60. Carter LG, D'Orazio JA, Pearson KJ. Resveratrol and cancer: focus on in vivo evidence. Endocr Relat Cancer 2014, 21(3): R209-225.

### [Example 2]

Murine colon adenocarcinoma MC38 cells (5 × 10⁵) were inoculated into C57BL/6 mice. After 7 days, anti-PD-L1 antibody (1-111A, 150 µg) and AMPK inhibitor (Compound C: 200 µg in BBS, 200 µL) were administered intraperitoneally. Anti-PD-L1 antibody was administered 3 times at a regular interval of 6 days; Compound C (C.C) was administered 7 times at a regular interval 2 days. Tumor volumes (mm³) from day 0 to day 25 of MC38 inoculation are shown.

The results are shown in Fig. 9. AMPK inhibitor enhances the efficacy of anti-PD-L1 antibody therapy.

### [Example 3]

Murine renal cell carcinoma RENCA cells (2 × 10⁶) were inoculated into BALB/c mice. After 7 days, anti-PD-L1 antibody (1-111A, 150 µg) and AMPK inhibitor (Compound C: 200 µg in BBS, 200 µl) were administered intraperitoneally. Anti-PD-L1 antibody was administered 3 times at a regular interval of 6 days; Compound C (C.C) was administered 7 times at a regular interval 2 days. Tumor volumes (mm³) from day 0 to day 25 of RENCA inoculation are shown.

The results are shown in Fig. 10. AMPK inhibitor enhances the efficacy of anti-PD-L1 antibody therapy.

### [Example 4]

MC38 cells (5 × 10⁵) were inoculated into C57BL/6 mice. After 7 days, anti-PD-L1 antibody (1-111A, 150 µg) and PGC-1α/transcription factor complex activator (2-(4- {2-[(4-chlorobenzoyl)amino]ethyl}phenoxy)-2-methylpropanoic acid (bezafibrate): 10 ug in PBS, 200 ul) were administered intraperitoneally. Anti-PD-L1 antibody was administered 3 times at a regular interval of 6 days; bezafibrate was administered 7 times at a regular interval 2 days. Tumor volumes (mm³) from day 0 to day 25 of MC38 inoculation are shown.

The results are shown in Fig. 11. PGC-1α/transcription factor complex activator enhances the efficacy of anti-PD-L1 antibody therapy.

### [Example 5]

MethA cells (5 × 10⁵) were inoculated into BALB/c mice. After 7 days, anti-PD-L1 antibody (1-111A, 80 µg) and Foxo1 inhibitor (5-amino-7-(cyclohexylamino)-1-ethyl-6-fluoro-4-oxo-1,4-dihydroquinoline-3-carboxylic acid, Calbiochem) (2mg/kg) were administered intraperitoneally. The administration schedule was as shown in Fig. 3. Tumor volumes (mm³) from day 0 to day 28 of MethA inoculation are shown in Fig. 16. Foxo1 inhibitor enhances the efficacy of anti-PD-L1 antibody therapy.

### [Example 6]

Amino acid contents in the sera of 5 each of PD-1^{-/-} mice [Immunity 11, 141-151 (1999); Science 291, 319-322 (2001); Nat. Med. 9, 1477-1483 (2003)] and wild-type C57BL/6N mice (in the same litter as PD-1^{-/-} mice) were determined by GC-MS analysis. The results are shown in Fig. 17. Fig. 17 shows the values for PD-1^{-/-} mice, with the values for wild-type mice being taken as 1. In PD-1 deficient mice, T cell division progresses and blood amino acids are metabolized, resulting in lower values thereof.

Fibrosarcoma MethA (Cell Resource Center for Biomedical Research) cells (5 × 10⁵) were inoculated into BALB/c mice. After 7 days, anti-PD-L1 antibody (1-111A, 60 µg) and Aminoleban (Otsuka Pharmaceutical, 400 µl/mouse) were administered. The administration schedule was as shown in Fig. 3. The composition of Aminoleban is shown in Fig. 18.

The results are shown in Fig. 19. Amino acids enhance the efficacy of anti-PD-L1 antibody therapy.

All publications, patents and patent applications cited herein are incorporated herein by reference in their entirety.

### INDUSTRIAL APPLICABILITY

The pharmaceutical composition of the present invention is applicable as an anticancer agent, a therapeutic for infections or a combination thereof.

### SEQUENCE LISTING FREE TEXT

### <SEQ ID NO: 1>

This sequence shows the nucleotide sequence of a forward primer.

### <SEQ ID NO: 2>

This sequence shows the nucleotide sequence of a reverse primer.

SEQUENCE TABLE

<110>  Kyoto University

<120>  Combinatorial therapies with PD-1 pathway inhibitors

<130>  FP-213PCT

<150>  JP 2015-238511
<151>  2015-12-07

<150>  JP 2016-119695
<151>  2016-06-16

<160>  2

<170>  PatentIn version 3.5

<210>  1
<211>  20
<212>  DNA
<213>  Artificial Sequence

<220>
<223>  primer

<400>  1
actcggattg ctccggccct                                                   20


<210>  2
<211>  22
<212>  DNA
<213>  Artificial Sequence

<220>
<223>  primer

<400>  2
actgacggcc taactccacc ca                                                22

The following numbered clauses, describing aspects of our proposals, are part of the description:
1. A pharmaceutical composition which comprises at least one substance selected from the group consisting of the following (i) to (iii) and is administered before, after or simultaneously with the administration of a PD-1 signal inhibitor:
   (i) ROS generators and substances that regulate downstream signaling pathways thereof,
   (ii) substances exhibiting an uncoupling effect and substances that regulate downstream signaling pathways thereof, and
   (iii) amino acids.
2. The pharmaceutical composition of clause 1, wherein the PD-1 signal inhibitor is an antibody.
3. The pharmaceutical composition of clauses 1 or 2, wherein the antibody is at least one antibody selected from the group consisting of anti-PD-1 antibody, anti-PD-L1 antibody and anti-PD-L2 antibody.
4. The pharmaceutical composition of any one of clauses 1 to 3, wherein the ROS generator is at least one compound selected from the group consisting of tert-butyl hydroperoxide, carbonyl cyanide p-trifluoromethoxyphenylhydrazone, 2,4-dinitrophenol, 2,3-dimethoxy-1, 4-naphthoquinone and analogs thereof.
5. The pharmaceutical composition of any one of clauses 1 to 3, wherein the substance exhibiting an uncoupling effect is at least one compound selected from the group consisting of carbonyl cyanide p-trifluoromethoxyphenylhydrazone, 2,4-dinitrophenol, carbonyl cyanide m-chlorophenylhydrazone, salicylic acid, 4,4'-[pentane-1,5-diylbis(oxy)]dibenzenecarboximidamide, 2-(2-(2,6-dichlorophenylamino)phenyl)acetic acid, 4-hydroxy-2-methyl-N-(2-pyridinyl)-2H-1,2-benzothiazine-3-carboxamide 1,1-dioxide, 2- {1-[(4-chlorophenyl)carbonyl]-5-methoxy-2-methyl-1H-indol-3-yl} acetic acid, N-(4-nitro-2-phenoxyphenyl)methanesulfonamide, 4-hydroxy-2-methyl-N-(5-methyl-2-thiazolyl)-2H-1,2-benzothiazine-3-carboxamide-1,1 -diox ide, niclosamide ethanolamine salt, 3-methylbut-2-enyl 4-methoxy-8-(3-methylbut-2-enyloxy)quinoline-2-carboxylate and analogs thereof.
6. The pharmaceutical composition of any one of clauses 1 to 3, wherein the substance that regulates downstream signaling pathways of ROS generators or substances exhibiting an uncoupling effect is a substance which regulates one or more of mTOR, AMPK, SIRT1, PGC-1α/transcription factor complex (PGC-1α-comprising transcription factor complex) and Foxo1.
7. The pharmaceutical composition of clause 6, wherein the substance that regulates mTOR is at least one compound selected from the group consisting of 4,6-di-4-morpholinyl-N-(4-nitrophenyl)-1,3,5-triazin-2-amine, phosphatidic acid and analogs thereof.
8. The pharmaceutical composition of clause 6, wherein the substance that regulates AMPK is at least one compound selected from the group consisting of 6,7-dihydro-4-hydroxy-3-(2'-hydroxy[1,1 '-biphenyl]-4-yl)-6-oxo-thieno[2,3-b]pyridine-5-car bonitrile, 5-aminoimidazole-4-carboxamide 1-β-D-ribofuranoside, N,N-dimethylimidodicarbonimidic diamide, 6-[4-[2-(1-piperidinyl)ethoxy]phenyl]-3-(4-pyridinyl)pyrazolo[1,5-a]pyrimidine and analogs thereof.
9. The pharmaceutical composition of clause 6, wherein the substance that regulates SIRT1 is at least one compound selected from the group consisting of trans-3,5,4'-trihydroxystilbene, N-(2-(3-(piperazin-1-ylmethyl)imidazo[2,1-b]thiazol-6-yl)phenyl)quinoxaline-2-carboxamide, N-benzyl-3,5-dicarbethoxy-4-phenyl-1,4-dihydropyridine, 2-amino-N-cyclopentyl-1-(3-methoxypropyl)-1H-pyrrolo[2,3-b]quinoxaline-3-carboxamide, nicotinamide mononucleotide and analogs thereof.
10. The pharmaceutical composition of clause 6, wherein the substance that regulates PGC-1α/transcription factor complex (transcriptional factor complexes comprising PGC-1 α ) is at least one compound selected from the group consisting of 2-(4- {2-[(4-chlorobenzoyl)amino]ethyl}phenoxy)-2-methylpropanoic acid, 9-cis,12-cis-octadecadienoic acid, 2-[4-(4-chlorobenzoyl)phenoxy]-2-methyl-propanoic acid-d6 1-methylethyl ester, (undecylthio)-acetic acid, 4-methyl-5 -(2-pyrazinyl)-3 -dithio lethione, N,N-dimethylformamide, 3-[4-(2,4-bis-trifluoromethylbenzyloxy)-3-methoxyphenyl]-2-cyano-n-(5-trifluoromethyl-1,3, 4-thiadiazol-2-yl)acrylamide and analogs thereof.
11. The pharmaceutical composition of clause 6, wherein the substance that regulates Foxo1 is at least one compound selected from the group consisting of 5-amino-7-(cyclohexylamino)-1-ethyl-6-fluoro-4-oxo-1,4-dihydroquinoline-3-carboxylic acid, The following numbered clauses, describing aspects of our proposals, are part of the description:
   2-cyclopentyl-N-{2,4-dichloro-3-[(isoquinolin-5-yloxy)methyl]phenyl}-N-methylacetamide and analogs thereof.
12. The pharmaceutical composition of any one of clauses 1 to 3, wherein the amino acid is at least one compound selected from the group consisting of tryptophan, phenylalanine, leucine, isoleucine, tyrosine, histidine, lysine, methionine, threonine, valine, alanine, arginine, asparagine, aspartic acid, cysteine, glutamic acid, glutamine, glycine, proline, serine, ornithine, citrulline and analogs thereof.
13. The pharmaceutical composition of any one of clauses 1 to 12, which is used as an anti-cancer agent, an anti-infective agent or a combination thereof.
14. The pharmaceutical composition of any one of clauses 1 to 13, wherein the PD-1 signal inhibitor is administered separately from the at least one substance selected from the group consisting of the following (i) to (iii):
   (i) ROS generators and substances that regulate downstream signaling pathways thereof,
   (ii) substances exhibiting an uncoupling effect and substances that regulate downstream signaling pathways thereof, and
   (iii) amino acids.
15. The pharmaceutical composition of any one of clauses 1 to 13, which is a combination drug comprising the PD-1 signal inhibitor and the at least one substance selected from the group consisting of the following (i) to (iii):
   (i) ROS generators and substances that regulate downstream signaling pathways thereof,
   (ii) substances exhibiting an uncoupling effect and substances that regulate downstream signaling pathways thereof, and
   (iii) amino acids.
16. A drug which enhances PD-1 signal inhibitory activity, comprising at least one substance selected from the group consisting of the following (i) to (iii):
   (i) ROS generators and substances that regulate downstream signaling pathways thereof,
   (ii) substances exhibiting an uncoupling effect and substances that regulate downstream signaling pathways thereof, and
   (iii) amino acids.
17. A method of treating cancer, infection or a combination thereof, comprising administering to a human or animal subject a pharmaceutically effective amount of at least one substance selected from the group consisting of the following (i) to (iii) before, after or simultaneously with the administration of a PD-1 signal inhibitor:
   (i) ROS generators and substances that regulate downstream signaling pathways thereof,
   (ii) substances exhibiting an uncoupling effect and substances that regulate downstream signaling pathways thereof, and
   (iii) amino acids.
18. Use of at least one substance selected from the group consisting of the following (i) to (iii) for treating cancer, infection or a combination thereof, wherein the at least one substance selected is administered before, after or simultaneously with the administration of a PD-1 signal inhibitor:
   (i) ROS generators and substances that regulate downstream signaling pathways thereof,
   (ii) substances exhibiting an uncoupling effect and substances that regulate downstream signaling pathways thereof, and
   (iii) amino acids.
19. Use of at least one substance selected from the group consisting of the following (i) to (iii) in a method for treating cancer, infection or a combination thereof, wherein the at least one substance selected is administered before, after or simultaneously with the administration of a PD-1 signal inhibitor:
   (i) ROS generators and substances that regulate downstream signaling pathways thereof,
   (ii) substances exhibiting an uncoupling effect and substances that regulate downstream signaling pathways thereof, and
   (iii) amino acids.

## Claims

1. A pharmaceutical composition which comprises a substance that regulates PGC-1α/transcription factor complex (PGC-1α-comprising transcription factor complex) for use in treating a cancer, an infection, or a combination thereof in a human subject in combination with a PD-1 signal inhibitor.

2. The pharmaceutical composition for use of claim 1, wherein the PD-1 signal inhibitor is an antibody.

3. The pharmaceutical composition for use of claim 1 or 2, wherein the antibody is at least one antibody selected from the group consisting of an anti-PD-1 antibody, an anti-PD-L1 antibody and an anti-PD-L2 antibody.

4. The pharmaceutical composition for use of any one of claims 1 to 3, wherein the substance that regulates PGC-1α/transcription factor complex (PGC-1α-comprising transcription factor complex) is at least one compound selected from the group consisting of 2-(4-{2-[(4-chlorobenzoyl)amino]ethyl}phenoxy)-2-methylpropanoic acid, 9-cis,12-cis-octadecadienoic acid, 2-[4-(4-chlorobenzoyl)phenoxy]-2-methyl-propanoic acid-d6 1-methylethyl ester, (undecylthio)-acetic acid, 4-methyl-5-(2-pyrazinyl)-3-dithiolethione, N,N-dimethylformamide, 3-[4-(2,4-bis-trifluoromethylbenzyloxy)-3-methoxyphenyl]-2-cyano-n-(5-trifluoromethyl-1,3,4-thiadiazol-2-yl)acrylamide and analogs thereof.

5. The pharmaceutical composition for use of any one of claims 1 to 4, which further comprises a substance which regulates one or more of mTOR, AMPK, SIRT1, a ROS generator and Foxo1.

6. The pharmaceutical composition for use of claim 5, wherein the substance that regulates mTOR is at least one compound selected from the group consisting of 4,6-di-4-morpholinyl-N-(4-nitrophenyl)-1,3,5-triazin-2-amine, phosphatidic acid and analogs thereof.

7. The pharmaceutical composition for use of claim 5, wherein the substance that regulates AMPK is at least one compound selected from the group consisting of 6,7-dihydro-4-hydroxy-3-(2'-hydroxy[1,1'-biphenyl]-4-yl)-6-oxo-thieno[2,3-b]pyridine-5-carbonitrile, 5-aminoimidazole-4-carboxamide 1-β-D-ribofuranoside, N,N-dimethylimidodicarbonimidic diamide, 6-[4-[2-(1-piperidinyl)ethoxy]phenyl]-3-(4-pyridinyl)pyrazolo[1,5-a]pyrimidine and analogs thereof.

8. The pharmaceutical composition for use of claim 5, wherein the substance that regulates SIRT1 is at least one compound selected from the group consisting of trans-3,5,4'-trihydroxystilbene, N-(2-(3-(piperazin-1-ylmethyl)imidazo[2,1-b]thiazol-6-yl)phenyl)quinoxaline-2-carboxamide, N-benzyl-3,5-dicarbethoxy-4-phenyl-1,4-dihydropyridine, 2-amino-N-cyclopentyl-1-(3-methoxypropyl)-1H-pyrrolo[2,3-b]quinoxaline-3-carboxamide, nicotinamide mononucleotide and analogs thereof.

9. The pharmaceutical composition for use of claim 5, wherein the ROS generator is at least one compound selected from the group consisting of tert-butyl hydroperoxide, carbonyl cyanide p-trifluoromethoxyphenylhydrazone, 2,4-dinitrophenol, 2,3-dimethoxy-1, 4-naphthoquinone and analogs thereof.

10. The pharmaceutical composition for use of claim 5, wherein the substance that regulates Foxo1 is at least one compound selected from the group consisting of 5-amino-7-(cyclohexylamino)-1-ethyl-6-fluoro-4-oxo-1,4-dihydroquinoline-3-carboxylic acid, 2-cyclopentyl-N-{2,4-dichloro-3-[(isoquinolin-5-yloxy)methyl]phenyl}-N-methylacetamide and analogs thereof.

11. The pharmaceutical composition for use of any one of claims 1 to 10, wherein the PD-1 signal inhibitor is administered separately from the at least one substance.

12. The pharmaceutical composition for use of any one of claims 1 to 10, wherein the PD-1 signal inhibitor and the at least one substance are formulated as a single dosage.

13. A pharmaceutical composition, comprising:
(a) a substance that regulates PGC-1α/transcription factor complex (PGC-1α-comprising transcription factor complex); and
(b) a PD-1 signal inhibitor.

14. The pharmaceutical composition of claim 13, further comprising:
(i) a substance that regulates mTOR comprising at least one compound selected from the group consisting of 4,6-di-4-morpholinyl-N-(4-nitrophenyl)-1,3,5-triazin-2-amine, phosphatidic acid and analogs thereof;
(ii) a substance that regulates AMPK comprising at least one compound selected from the group consisting of 6,7-dihydro-4-hydroxy-3-(2'-hydroxy[1,1'-biphenyl]-4-yl)-6-oxo-thieno[2,3-b]pyridine-5-carbonitrile, 5-aminoimidazole-4-carboxamide 1-β-D-ribofuranoside, N,N-dimethylimidodicarbonimidic diamide, 6-[4-[2-(1-piperidinyl)ethoxy]phenyl]-3-(4-pyridinyl)pyrazolo[1,5-a]pyrimidine and analogs thereof;
(iii) a substance that regulates SIRT1 comprising at least one compound selected from the group consisting of trans-3,5,4'-trihydroxystilbene, N-(2-(3-(piperazin-1-ylmethyl)imidazo[2,1-b]thiazol-6-yl)phenyl)quinoxaline-2-carboxamide, N-benzyl-3,5-dicarbethoxy-4-phenyl-1,4-dihydropyridine, 2-amino-N-cyclopentyl-1-(3 -methoxypropyl)-1H-pyrrolo[2,3-b]quinoxaline-3-carboxamide, nicotinamide mononucleotide and analogs thereof;
(iv) a ROS generator comprising at least one compound selected from the group consisting of tert-butyl hydroperoxide, carbonyl cyanide p-trifluoromethoxyphenylhydrazone, 2,4-dinitrophenol, 2,3-dimethoxy-1, 4-naphthoquinone and analogs thereof; or
(v) a substance that regulates Foxo1 comprising at least one compound selected from the group consisting of 5-amino-7-(cyclohexylamino)-1-ethyl-6-fluoro-4-oxo-1,4-dihydroquinoline-3-carboxylic acid, 2-cyclopentyl-N-{2,4-dichloro-3-[(isoquinolin-5-yloxy)methyl]phenyl}-N-methylacetamide and analogs thereof.

15. The pharmaceutical composition of claim 13 or 14, wherein the PD-1 signal inhibitor is an antibody.
